# EUROPEAN PATENT APPLICATION

(11) **EP 4 763 223 A1**
(43) Date of publication of application: **24.06.2026**
(21) Application number: 24223099.3
(22) Date of filing: 23.12.2024
(51) Int. Cl.: A61K 40/11, A61K 40/42

(54) **AN ENGINEERED CELL COMPRISING A CHIMERIC ANTIGEN RECEPTOR AND A SWITCHABLE CHIMERIC ANTIGEN RECEPTOR, A KIT AND THEIR USE**

(71) Applicant: AvenCell Therapeutics Inc., Watertown, MA 02472 (US)
(72) Inventor: Boyerinas, Ben, Watertown (US); Spehr, Johannes, Dresden (DE); Ashoura, Norah, Watertown (US); Kuiper, Emiliy, Watertown (US)
(74) Representative: Kailuweit & Uhlemann Patentanwälte Partnerschaft mbB

(57) **Abstract**

The present invention relates to an engineered cell comprising at least one chimeric antigen receptor (CAR) polypeptide and a switchable CAR in particular for use in the treatment of cancer, infectious disease or autoimmune disease, a pharmaceutical composition comprising the engineered cell and a kit comprising the engineered cell or at least two nucleic acids and/or vectors comprising a nucleotide sequence encoding the at least one CAR polypeptide or comprising a nucleotide sequence encoding the at least one switchable CAR polypeptide.

## Description

The present invention relates to an engineered cell comprising at least one chimeric antigen receptor (CAR) polypeptide and a switchable CAR in particular for use in the treatment of cancer, infectious disease or autoimmune disease, a pharmaceutical composition comprising the engineered cell and a kit comprising the engineered cell or at least two nucleic acids and/or vectors comprising a nucleotide sequence encoding the at least one CAR polypeptide or comprising a nucleotide sequence encoding the at least one switchable CAR polypeptide.

The adoptive transfer of the genetically engineered immune effector cells targeted to bind to target cells through chimeric antigen receptors (CARs) aims to rapidly establish a T-cell mediated disease cell response. When expressed in T-cells, CARs efficiently redirect T-cell specificity and cytotoxicity e. g. to tumor cells. Through this approach, CAR-T cells overcome issues with immune tolerance and the requirement of major histocompatibility complex (MHC) presentation of target cells.

Chimeric antigen receptors (CARs) are artificial receptors consisting of a binding moiety, which provides the antigen-specificity, e. g. a single chain variable fragment (scFv) recognizing a surface antigen of a tumor cell, a transmembrane domain, and one or several signaling chains derived from immune receptors, e. g. CD3ζ, that activate an immune cell (Cartellieri et al. 2010).

The first-generation CAR comprising one signaling chain was modified by adding a signal transduction domain of e. g. CD28 or CD137 (4-1 BB), which are co-stimulatory molecules of a T cell, to increase the activation of immune cells (second generation CAR). A third generation CAR was developed by tandemly linking an additional complementary signal transduction domain derived from e. g. CD28, CD137 (4-1 BB) or CD134 (OX40), both which are tumor necrosis factor (TNF) receptor superfamily members, to a second-generation CAR.

Immune cells, genetically modified to express CARs, can be used to bind cells or tissue structures expressing the appropriate target of the CAR binding moiety. Cross-linking leads to an induction of signal pathways via the CAR signaling chains, which will change the biologic properties of the CAR-engrafted immune cell. CAR activation in gene-modified conventional T cells induces the release of inflammatory cytokines, such as interferon-gamma (IFN-g), and promotes cytotoxicity towards target antigen-expressing cells. In contrast, CAR activation in gene-modified regulatory T cells (Tregs) leads to an activation of Treg-specific immunomodulatory and suppressive mechanisms like interleukin (IL)-10 or tumor growth factor-beta (TGF-β) secretion. The adoptive transfer of immune cells engineered with CARs is currently considered a highly promising therapeutic option for the treatment of otherwise incurable malignant, infectious or autoimmune diseases.

Clinical trials demonstrated both the safety and the feasibility of this treatment strategy (Lamers et al. 2006, Kershaw et al. 2006). In further trials, a majority of patients suffering from late-stage tumors of B cell origin showed complete or at least partial response to a treatment with autologous T cells equipped with a CD19-specific CAR, which lasted for several months (Brentjens et al. 2011, Grupp et al. 2013, Kalos et al. 2011, Kobos et al. 2013).

Until today, four CAR-T cell therapeutics have gained market approval for treatment of B cell derived malignancies, proving the clinical feasibility of this approach.

However, the conventional CAR technology comes along with some critical issues. Unexpected target gene expression on normal tissue may provoke a rapid and rigorous immune reaction of engineered T cells against normal cells, which can cause severe side effects (Morgan et al. 2010). Another drawback of conventional CAR technology is the restriction of engineered T cell retargeting to a single antigen. Such a monotherapeutic approach implies the risk for the development of tumor escape variants, which have lost the target antigen during treatment. The emergence of tumor escape variants under conventional CAR-T cell therapy after several months was already observed in clinical trials (Sotillo et al. 2015).

Modular switchable "universal" CAR T (UniCAR) approaches can overcome these limitations by separating antigen recognition and activating domain of a CAR into two separate operational units. T cells are engineered to express a CAR with a universal binding domain recognizing a tag (Cartellieri et al. 2016). Antigen-specificity is provided by soluble adapter molecules, which consist of an antigen-binding domain fused to the tag recognized by the UniCAR. Cartellieri et al. describe the treatment of CD33- and/or CD123-positive acute myeloid leukemia cells *in vitro* and *in vivo.* Next to the UniCAR approach for recognizing various antigens (EP 2 990 416 A1) a reversed universal CAR (RevCAR) approach is known that promotes binding of an immune cell engineered to express a RevCAR comprising a tag to a target cell through an adaptor molecule comprising a tag-binding domain and a target cell binding domain (EP 3 581 200 A1).

Moreover, switchable CAR T approaches like UniCAR or RevCAR provide the possibility to rest CAR-T cells in-between cycles of activation and stimulation by pausing administration of the soluble targeting module molecule. This is expected to prevent the exhaustion observed upon continuous stimulation of conventional CAR-T cells thereby improving persistence (Weber et al. 2021).

WO 2016/154621 A1 describes switchable chimeric receptors comprising a non-antibody extracellular domain that interacts with a chimeric receptor binding partner presented on a switch. Furthermore, the chimeric receptor comprises a transmembrane domain and an intracellular signaling domain. The switch comprises the chimeric receptor binding partner, preferably an antibody or antibody fragment, and a targeting moiety. Preferably the chimeric receptor binding peptide comprises a peptide epitope tag. Preferably the chimeric receptor binding partner and the non-antibody extracellular domain are selected from the group consisting of isopeptag and pilin, spytag and spycatcher, SNARE and SNAP25, synaptobrevin and syntaxin, Hu-tag and RNAse I, K4 peptide and E4 peptide or a modified K4 peptide and a modified E4 peptide, a first mouse coronin 1A alpha helix and a second mouse coronin 1A alpha helix, an anchor domain of an A-kinase anchor protein and a regulatory subunit of cAMP-dependent protein kinase A (AD) and a dimerization and docking domain of cAMP-dependent protein kinase (DDD), barnase and barstar, coronin and ccCor1 peptide. The targeting moiety may target an antigen selected from CD19, Her2, CLL-1, CD33, EGFRvlll, CD20, CD22, BCMA or a fragment thereof.

Darowski et al. describe flexible CAR adaptor molecules for the recruitment of CAR-T cells with tags like 5B9, GCN4, FITC, leucine zipper sequences, or biotinylated IgG, and targets like CD33, CD123, CD19, CD20, CD22, HER2, EGFR, CCR4, G2D, MCSP, ErbB2 (Darowski et al. 2019).

Feldmann et al. disclose RevCAR-T cells efficiently killing tumor cells, flexibly redirected against multiple targets by exchanging the targeting modules, in particular targeting modules against PSMA and PSCA (Feldmann et al. 2020).

Alternatively, a method for use in the case of tumors with a heterogeneous antigenic profile and/or the formation of tumor escape variants is the targeting of more than one antigen. US 2018/0187149 A1 describes compositions and methods relating to CAR polypeptides and engineered cells having CAR polypeptides directed to at least two different targets, wherein preferably the target of the first and the second antigen recognition domain are selected from the group consisting of interleukin 6 receptor, NY-ESO-1, alpha fetoprotein (AFP), glypican-3 (GPC3), BCMA, BAFF-R, BCMA, TAC1, LeY, CD5, CD13, CD14, CD15, CD19, CD20, CD22, CD33, CD41, CD61, CD64, CD68, CD117, CD123, CD138, CD267, CD269, CD38, Flt3 receptor, and CS1.

The task is therefore to provide an engineered cell comprising a chimeric antigen receptor for the treatment of cancer, infectious disease or autoimmune disease with improved immune response and/or reduced risk for relapse.

According to the invention, the task is solved by engineered cells, a pharmaceutical composition and a kit according to the independent claims. Advantageous embodiments of the invention are indicated in the dependent claims.

A first aspect of the invention is an engineered cell comprising:
a) at least one chimeric antigen receptor (CAR) polypeptide comprising:
   - at least one target cell-binding domain,
   - a first extracellular hinge domain,
   - a first transmembrane domain, and
   - at least one first intracellular signaling domain,
b) at least one switchable chimeric antigen receptor (CAR) polypeptide comprising:
   - a tag-binding domain or a tag,
   - a second extracellular hinge domain,
   - a second transmembrane domain, and
   - at least one second intracellular signaling domain.

Advantageously, the engineered cell according to the invention targets at least two antigens simultaneously, in particular directly by the at least one target cell-binding domain and indirectly by the tag-binding domain or tag, which binds to a targeting module comprising a further target cell-binding domain. This combination of the specificity of the CAR and the switchable CAR on the cell embodies a significant advantage for treating tumors with a heterogeneous antigenic profile or for preventing relapse following loss of target antigen expression.

Advantageously, the engineered cell according to the invention administered with a targeting module could delay tumor growth, wherein the anti-tumor response of the switchable chimeric antigen receptor is only induced in the presence of the targeting module. The effect can be interrupted by withholding the administration of the targeting module to rest the switchable CAR-T cells and prevent their exhaustion. The switchable CAR-T cells can be reactivated and reexpanded by additional targeting module doses.

As used herein, the term "targeting module" refers to a molecule, preferably a polypeptide or protein with at least three two domains, wherein each domain is specific for a target or a uniform group of targets, respectively, wherein at least one domain is specific for a target cell, in particular the target cell-binding domain; and one domain is specific for a switchable chimeric antigen receptor, in particular the tag-binding domain or tag.

Suitably, the target cell-binding domain of the CAR polypeptide and the target cell-binding domain of the targeting module are different.

In embodiments, the engineered cell is isolated. As used herein, the term "isolated" means altered or removed from the natural state.

As used herein, the term "chimeric antigen receptor" refers to an artificial chimeric fusion protein, in particular a receptor comprising an antigen-binding domain, an extracellular hinge and a transmembrane domain and a signal transduction domain (intracellular signaling domain). The domains can be derived from different sources and therefore, the receptor is called chimeric.

As used herein, the term "switchable chimeric antigen receptor" refers to an artificial chimeric fusion protein, in particular a receptor comprising a tag-binding domain or a tag, an extracellular hinge and a transmembrane domain and a signal transduction domain. The domains can be derived from different sources and therefore, the receptor is called chimeric. Advantageously, the receptor can bind with the tag-binding domain or tag to different targeting modules.

Advantageously, the cell comprising a nucleotide sequence encoding a switchable CAR expresses the switchable CAR, which has binding specificity for the tag or tag-binding domain of the targeting module, which in turn binds to an antigen on a target cell.

As used herein, the term "domain" refers to a part of a protein sequence, which can exist and function independently from the rest of the protein.

As used herein, the term "target cell-binding domain" refers to a peptide, protein, or low molecular weight organic ligand, which specifically binds a protein or protein complex (antigen) on the surface of a target cell, in particular a cancer cell, T cell, infected cell, pathogens or parasites.

As used herein, the terms "peptide", "polypeptide" and "protein" are used interchangeably and refer to a compound comprised of amino acid residues covalently linked by peptide bonds. A protein or peptide must contain at least two amino acids, and no limitation is placed on the maximum number of amino acids that can comprise a protein's or peptide's sequence.

As used herein, the term "low molecular weight organic ligand" refers to an organic molecule with a molecular weight of maximal 10 kilodaltons, preferably of maximal 3 kilodaltons, which specifically binds a protein or protein complex (antigen) on the surface of a target cell, preferably a cancer cell, T cell, infected cell or pathogens or parasites.

In embodiments, the target cell-binding domain can also be selected from soluble T cell receptors, which are composed of the alpha and beta or the gamma and delta chains of a T cell receptor (TCR), fragments or mutants thereof. Such TCR-derived binding moieties recognize and bind to peptides presented by human leukocyte antigen class (HLA) I and II protein complexes. Examples are, but are not limited to, TCRs specific for peptides derived from proteins like EGFR family, survivin, sry-like high motility group box (SOX) protein family, melanoma-associated antigens (e.g. auto immunogenic cancer/testis antigen NY-ESO-1, members of the melanoma antigen family A MAGEA, the preferentially expressed antigen in melanoma PRAME), and leukemia-associated antigens (e.g. Wilms tumor gene 1 WT1).

In embodiments, the target cell-binding domain is a soluble T cell receptor consisting of the alpha and beta or the gamma and delta chain of a T cell receptor (TCR).

In embodiments, the target cell-binding domain is an antibody, an antibody fragment, a protein, a peptide or a low molecular weight organic ligand, preferably a peptide, an antibody or an antibody fragment. In embodiments, the further antigen-binding domain is an antibody fragment. In embodiments, the target cell-binding domain is selected from single-chain variable fragments (scFv), single-chain antibodies, Fc fragment, F(ab')2 fragments, Fab fragments, and fragments produced by a Fab expression library or single-domain antibodies (nanobodies).

In embodiments, the target cell-binding domain is specific for at least one surface antigen selected from the group consisting of CD2, CD3, CD4, CD5, CD7, CD8, CD10, CD15, CD19, CD20, CD22, CD23, CD25, CD30, CD33, CD38, CD44, CD44v6, CD52, CD66a, CD66b, CD66c, CD66d, CD66e, CD66f, CD79a, CD79b, CD90, CD99, CD123, CD133, CD135, CD150, CD181, CD182, CD184, CD223, CD229, CD269, CD273, CD274, CD276, CD279, CD319, CD366, CD371, a cytokine receptor, CXCR4, c-Met, mesothelin, a member of the epidermal growth factor receptor family or a mutant thereof, a member of the tumor necrosis factor receptor superfamily, a claudin, an ephrin, an ephrin receptor, a fucosyl transferase, a prostate specific antigen, an embryonic antigen, a member of the vascular endothelia growth factor family, epithelial cell adhesion molecule, alpha-fetoprotein, a member of the intercellular adhesion molecule family, a C-type lectin, an integrin, a member of the mucin protein family, a follicle-stimulating hormone receptor, a high molecular weight-melanoma associated antigen, a folate binding protein, a folate receptor, a somatostatin receptor, a ligand of the NKG2D receptor, a member of the epithelia glycoprotein family, a disialoganglioside, a glypican, a G protein-coupled receptor, a human papillomavirus protein, cancer/testis antigen, fibroblast activation protein, a member of the carbonic anhydrase family, a member of the carbohydrate antigen family, a Notch ligand, melanoma-associated chondroitin sulfate proteoglycan, glycoprotein A33, guanylate cyclase 2C and tumor-specific glycan.

In embodiments, the target cell-binding domain is specific for at least one surface antigen selected from the group consisting of CD2, CD3, CD4, CD5, CD7, CD8, CD10, CD15, CD19, CD20, CD22, CD23, CD25, CD30, CD33, CD38, CD44, CD44v6, CD52, CD66a, CD66b, CD66c, CD66d, CD66e, CD66f, CD79a, CD79b, CD90, CD99, CD123, CD133, CD135, CD150, CD181, CD182, CD184, CD223, CD229, CD269, CD273, CD274, CD276, CD279, CD319, CD366, CD371, a cytokine receptor, CXCR4, c-Met, mesothelin, a member of the epidermal growth factor receptor family, a member of the tumor necrosis factor receptor superfamily, a claudin, an ephrin, an ephrin receptor, a fucosyl transferase, a prostate specific antigen, an embryonic antigen, a member of the vascular endothelia growth factor family, epithelial cell adhesion molecule, alpha-fetoprotein, a member of the intercellular adhesion molecule family, a C-type lectin, an integrin, a member of the mucin protein family, a follicle-stimulating hormone receptor, a high molecular weight-melanoma associated antigen, a folate binding protein, a folate receptor, a somatostatin receptor, a ligand of the NKG2D receptor, a member of the epithelia glycoprotein family, a disialoganglioside, a glypican, a G protein-coupled receptor, a human papillomavirus protein, cancer/testis antigen, fibroblast activation protein, a member of the carbonic anhydrase family, a member of the carbohydrate antigen family, a Notch ligand, melanoma-associated chondroitin sulfate proteoglycan, glycoprotein A33, guanylate cyclase 2C and tumor-specific glycan.

In embodiments, the target cell-binding domain is specific for at least one surface antigen selected from the group consisting of CD2, CD3, CD4, CD5, CD7, CD8, CD10, CD15, CD19, CD20, CD22, CD23, CD25, CD30, CD33, CD38, CD44, CD44v6, CD52, CD66a, CD66b, CD66c, CD66d, CD66e, CD66f, CD79a, CD79b, CD90, CD99, CD123, CD133, CD135, CD150, CD181, CD182, CD184, CD223, CD229, CD269, CD273, CD274, CD276, CD279, CD319, CD366, CD371, CXCR4, c-Met, mesothelin, a claudin, an ephrin, an ephrin receptor, a fucosyl transferase, a prostate specific antigen, an embryonic antigen, epithelial cell adhesion molecule, alpha-fetoprotein, a C-type lectin, a follicle-stimulating hormone receptor, a high molecular weight-melanoma associated antigen, a folate binding protein, a folate receptor, a somatostatin receptor, a ligand of the NKG2D receptor, a disialoganglioside, a glypican, a G protein-coupled receptor, a human papillomavirus protein, cancer/testis antigen, fibroblast activation protein, a Notch ligand, melanoma-associated chondroitin sulfate proteoglycan, glycoprotein A33, and guanylate cyclase 2C.

In embodiments, the at least one target cell-binding domain is an antibody fragment that binds to CD19, CD20, CD123, IL13Rα2, HER-2, PD-L1 and/or PD-L2.

In embodiments, the at least one target cell-binding domain is specific for CD19 and/or CD20 and comprises at least one CD19-binding domain and/or at least one CD20-binding domain. In embodiments, the at least one target cell-binding domain is specific for CD19 and CD20 and comprises at least one CD19-binding domain and at least one CD20-binding domain. Advantageously, the at least trispecific engineered cell targets both CD19 and CD20 simultaneously.

As used herein "CD19" (Cluster of Differentiation 19) refers to a surface antigen expressed by B lymphocytes.

As used herein "CD20" (Cluster of Differentiation 20) refers to a glycosylated phosphoprotein expressed on the membrane surface of B cells.

In embodiments, the at least one CD19-binding domain comprises a heavy chain variable region having complementary-determining regions (CDRs) with an amino acid sequence according to SEQ ID No. 1 (CDR-H1), an amino acid sequence according to SEQ ID No. 2 (CDR-H2), and an amino acid sequence according to SEQ ID No. 3 (CDR-H3), and a light chain variable region having CDRs with an amino acid sequence according to SEQ ID No. 4 (CDR-L1), an amino acid sequence HTS (His-Thr-Ser) (CDR-L2), and an amino acid sequence according to SEQ ID No. 5 (CDR-L3).

In embodiments, the at least one CD19-binding domain comprises a sequence with an identity of at least 95 % with the amino acid sequence according to SEQ ID No. 6.

In embodiments, the at least one CD20-binding domain comprises a heavy chain variable region having CDRs with an amino acid sequence according to SEQ ID No. 7 (CDR-H1), an amino acid sequence according to sequence SEQ ID No. 8 (CDR-H2), and an amino acid sequence according to SEQ ID No. 9 (CDR-H3).

In embodiments, the at least one CD20-binding domain comprises a sequence with an identity of at least 95 % with the amino acid sequence according to SEQ ID No. 10.

As used herein, the term "heavy chain variable region (V_{H})" refers to the N-terminal variable region of the heavy chain of an antibody. As used herein, the term "light chain variable region (V_{L})" refers to the N-terminal variable region of the light chain of an antibody.

As used herein, the term "CDR (Complementarity-determining regions)" refers to parts of the variable chains in antibodies or antibody fragments, where the antibodies or antibody fragments bind to their specific antigen. An antibody comprises three CDRs (CDR1, CDR2 and CDR3), arranged non-consecutively, on the amino acid sequence of each variable domain and thus, six CDRs on the two variable domains (V_{H} and V_{L}), which can come into contact with the antigen. A single-domain antibody (sdAb), e.g. a V_{H}H fragment, comprises three CDRs (CDR1, CDR2 and CDR3), arranged non-consecutively, on the amino acid sequence of its variable domain, which can come into contact with the antigen.

As used herein, the term "specific" refers to the ability of an antibody or antibody fragment or a protein, peptide or low molecular weight organic ligand to recognize and bind with a binding partner (e.g. a tumor antigen) present in a sample, but not substantially recognize or bind other molecules in the sample.

As used herein, the term "binds" or "binding" refers to a non-covalent binding, in particular ionic bonds, hydrogen bonds, Van der Waals forces and/or hydrophobic interactions.

As used herein, the term "antibody" refers to a protein, which binds antigens via the antigen-binding fragment variable region (Fab). This is composed of one constant and one variable domain of each of the heavy (V_{H}) and the light chain (V_{L}). As used herein, the term "antibody fragment" or "antigen-binding fragment" refers to a protein comprising at least the V_{L} or V_{H} of an antibody. In embodiments, antibody fragments are selected from single-chain variable fragments (scFv), single-chain antibodies, Fc fragment, F(ab')2 fragments, Fab fragments, and fragments produced by a Fab expression library or single-domain antibodies (nanobodies).

As used herein, the term "single-chain variable fragment (scFv)" refers to an artificial antibody fragment comprising a variable domain of a light chain (V_{L}) and a variable domain of a heavy chain (V_{H}) of an antibody covalently linked. In embodiments, the V_{L} and V_{H} of an antibody are covalently linked by a short peptide of 10 to 25 amino acids. In further embodiments, the short peptide links the N-terminus of the V_{H} with the C-terminus of the V_{L}, or vice versa.

As used herein, the term "Fab fragments" refers to an antibody fragment comprising one constant and one variable domain of each of the heavy and the light chain.

As used herein, the term "single-domain antibody (sdAb)" or "nanobody" is an antibody fragment consisting of a single monomeric variable antibody domain, in particular a V_{H}H fragment.

In embodiments, the antibody is obtained from an animal species, preferably from a mammal such as human, simian, mouse, rat, rabbit, lama, alpaca, camel, guinea pig, horse, cow, sheep, goat, pig, dog or cat. Preferably, the antibody or antibody fragment is a human, humanized or deimmunized antibody. Humanized antibodies can be prepared in various ways, for example, by resurfacing and CDR grafting. In case of resurfacing, a combination of molecular modeling, statistical analyses, and mutagenesis is used to modify all non-CDR regions on the surface of the antibody to become similar to the surface of antibodies of the target organism. In CDR grafting, the CDR regions are introduced into known human framework regions, which are similar in sequence to the original ones. Deimmunized antibodies can be obtained by specifically mutating residues that confer immunogenicity hotspots as predicted based on in silico peptide-MHC affinity prediction.

In embodiments, the antibody or antibody fragment is a polyclonal, a monoclonal or a chimeric antibody, wherein an antigen-binding region of a non-human antibody is transferred into the framework of a human antibody by recombinant DNA techniques including in silico design.

In embodiments, antibodies to a selected antigen may be produced by immunization of various hosts including, but not limited to, goats, rabbits, rats, mice, through injection with cells expressing a particular protein, DNA or RNA encoding for the protein, the protein itself or any portion, fragment or oligopeptide that retain immunogenic properties of the protein.

In embodiments, the CD19-binding domain and/or the CD20-binding domain are independently selected from antibody and antibody fragment.

In embodiments, the at least one CD19-binding domain and/or the CD20-binding domain are antibody fragments.

In embodiments, the CD19-binding domain and/or the CD20-binding domain are independently selected from single-chain variable fragments (scFv), single-chain antibodies, Fc fragment, F(ab')2 fragments, Fab fragments, and fragments produced by a Fab expression library or single-domain antibodies (nanobodies), in particular a V_{H}H fragments.

In embodiments, both the CD19-binding domain and the CD20-binding domain are V_{H}H fragments.

In embodiments, the CD19-binding domain is a V_{H}H. In embodiments, the CD20-binding domain is an scFv fragment.

In preferred embodiments, the CD19-binding domain is an scFv. In preferred embodiments, the CD20-binding domain is a V_{H}H fragment.

In embodiments, the variable region(s) of the at least one CD19-binding domain and/or at least one CD20-binding domain comprise(s) a humanized amino acid sequence.

As used herein, the term "% sequence identity" refers to the number of identical amino acid residues in relation to the length of the amino acid sequence. Suitably, the percentage of identity of amino acid sequences between two sequences refers to an alignment over the whole length of the two sequences compared to each other and is obtained by using the EM-BOSS Water Pairwise Sequence Alignments (protein) programme (https://www.ebi.ac.uk/jdispatcher/ psa/emboss_water) for amino acid sequences. Those tools provided by the European Molecular Biology Laboratory (EMBL) European Bioinformatics Institute (EBI) for local sequence alignments use a modified Smith-Waterman algorithm (see https://www.ebi.ac.uk/jdispatcher/psa and Smith and Waterman 1981). When conducting an alignment, the default parameters defined by the EMBL-EBI are used. Those parameters are for amino acid sequences: Matrix = BLOSUM62, gap open penalty = 10 and gap extend penalty = 0.5.

In embodiments, the CD19-binding domain comprises an amino acid sequence with an identity of at least 98 %, preferably at least 99 %, with the amino acid sequence according to SEQ ID No. 6.

In embodiments, the CD20-binding domain comprises an amino acid sequence with an identity of at least 98 %, preferably at least 99 %, with the amino acid sequence according to SEQ ID No. 10.

In embodiments, the CD19-binding domain comprises an amino acid sequence with at least 99 % sequence similarity with the amino acid sequence according to SEQ ID No. 6.

In embodiments, the CD20-binding domain comprises an amino acid sequence with at least 99 % sequence similarity with the amino acid sequence according to SEQ ID No. 10.

As used herein, the term "similarity" refers to conservative substitutions of amino acid residues having similar physicochemical properties over the length of the amino acid sequence are comprised. In embodiments, the term "similarity" also comprises amino acid sequences with amino acids comprising modifications selected from the group comprising D amino acids, pseudo peptide bonds, amino alcohols, amino acids with modified side chains, in particular cysteine and selenocysteine, and/or circular proteins. Advantageously, said amino acids reveal increased stability of the single domain antibody. The % sequence similarity is determined with any reasonable similarity-scoring matrix known by the person skilled in the art, preferably with a similarity-scoring matrix selected from BLOSUM50, BLOSUM62, PMBEC orVTML10 to VTML80.

In embodiments, the at least one CD19-binding domain comprises the amino acid sequence according to SEQ ID No. 6.

In embodiments, the at least one CD20-binding domain comprises the amino acid sequence according to SEQ ID No. 10.

In embodiments, the different domains of the chimeric antigen receptor polypeptide are linked with each other by a linker. As used herein, the term "linker" (also spacer) refers to a molecule or molecule part separating at least two elements under consideration, in particular selected from functional groups, binding domains or binding domain subunits, such as a V_{L} and a V_{H} domain. Advantageously, the linker is a flexible peptide sequence that is selected such that the domains have a three-dimensional folding that allows them to exhibit their specificity. Moreover advantageously, the linker increases the protease resistance of the polypeptides.

In embodiments, the linker is a glycine-serine linker with the structure GₓS_{y}, wherein x and y are selected from 1 to 10, preferably 3 to 5. In embodiments, the linker comprises 1 to 10 repeats of the sequence G₄S₁ (SEQ ID No. 11).

In embodiments, the linker comprises 20 to 30 amino acids, preferably 25 amino acids.

In embodiments, the linker is an amino acid sequence according to SEQ ID No. 12, SEQ ID No. 13 or SEQ ID No. 14.

In embodiments, the linker between the V_{L} and V_{H} domain of the CD19-binding domain is an amino acid sequence according to SEQ ID No. 14.

In embodiments, the linker between the CD19-binding domain and the CD20-binding domain is an amino acid sequence according to SEQ ID No. 12 or SEQ ID No. 13, preferably according to SEQ ID No. 13.

In embodiments, the switchable CAR polypeptide is a reversible CAR (RevCAR) polypeptide comprising a tag, an extracellular hinge domain, a transmembrane domain and at least one intracellular signaling domain.

As used herein, the term "tag" refers to a marker, in particular a peptide sequence or an organic molecule, attached to peptides or proteins to enable them to bind to specific atoms, ions or molecules, in particular the tag-binding domain.

In embodiments, the tag is selected from organic molecules including fluorescence labels, e.g., FITC (Fluorescein isothiocyanate), and biotin.

In embodiments, the tag is a peptide epitope tag. In further embodiments, the tag comprises 10 to 20 amino acids.

In embodiments, the peptide epitope tag is a myc-tag, a His-tag, a peptide sequence from yeast transcription factor GCN4, preferably according to SEQ ID No. 15, SEQ ID No. 16 or mutants thereof; a leucine zipper sequence, preferably SYNZIP 1 to SYNZIP 48, BATF, FOS, ATF4, ATF3, BACH1, JUND, NFE2L3, HEPTAD (Reinke et al. 2010), a sequence according to SEQ ID No. 17 or SEQ ID No. 18 or mutants thereof; or a peptide sequence from a human nuclear protein. In embodiments, the peptide epitope tag is a myc-tag, a His-tag, a peptide sequence from yeast transcription factor GCN4 according to SEQ ID No. 15, SEQ ID No. 16, a leucine zipper sequence selected from SYNZIP 1 to SYNZIP 48, BATF, FOS, ATF4, ATF3, BACH1, JUND, NFE2L3, HEPTAD (Reinke et al. 2010), a sequence according to SEQ ID No. 17 or SEQ ID No. 18 or a peptide sequence from a human nuclear protein.

In embodiments, the tag is a peptide sequence from the human La protein, in particular according to SEQ ID No. 19, SEQ ID No. 20, SEQ ID No. 21 or mutants thereof.

In embodiments, the tag is the human La epitope E5B9 according to SEQ ID No. 19 or E7B6 according to SEQ ID No. 20 or SEQ ID No. 21, preferably the human La epitope E5B9 according to SEQ ID No. 19 or E7B6 according to SEQ ID No. 21.

As used herein, the term "nuclear protein" refers to a protein found in the cell nucleus. Advantageously, tags, which are peptide sequences from nuclear antigens, cannot be accessed and bound by the corresponding tag-binding domain in the context of the native protein under physiological conditions. Further advantageously, the tag is not immunogenic. This leads to minimization of the risk of uncontrolled on-target off-site toxicities by CAR-expressing immune cells like the release of toxic levels of cytokines, referred to variously as cytokine storms or cytokine release syndrome (CRS).

In embodiments, the His-tag is an amino acid sequence consisting of histidine residues, preferably in the range of six to fourteen histidine residues.

In embodiments, the switchable CAR polypeptide is a UniCAR polypeptide comprising a tag-binding domain, an extracellular hinge domain, a transmembrane domain and at least one intracellular signaling domain.

In embodiments, the tag-binding domain is an antibody or antigen-binding fragment.

In embodiments, the tag-binding domain is an antibody or an antigen-binding fragment specific for a myc-tag, a His-tag, a peptide sequence from yeast transcription factor GCN4, preferably according to SEQ ID No. 15, SEQ ID No. 16 or mutants thereof; a leucine zipper sequence, preferably SYNZIP 1 to SYNZIP 48, BATF, FOS, ATF4, ATF3, BACH1, JUND, NFE2L3, HEPTAD (Reinke et al. 2010), a sequence according to SEQ ID No. 17 or SEQ ID No. 18 or mutants thereof; or a peptide sequence from a human nuclear protein. In embodiments, the tag-binding domain is an antibody or an antigen-binding fragment binding to a myc-tag, a His-tag, a peptide sequence from yeast transcription factor GCN4 according to SEQ ID No. 15, SEQ ID No. 16, a leucine zipper sequence selected from SYNZIP 1 to SYNZIP 48, BATF, FOS, ATF4, ATF3, BACH1, JUND, NFE2L3, HEPTAD (Reinke et al. 2010), a sequence according to SEQ ID No. 17 or SEQ ID No. 18 or a peptide sequence from a human nuclear protein.

In embodiments, the tag-binding domain is an antigen-binding fragment. In embodiments, the tag-binding domain is an scFv or a Fab fragment.

In embodiments, the tag-binding domain is an scFv binding a La epitope. In embodiments, the tag-binding domain is an scFv binding La epitope 5B9 according to SEQ ID No. 19 or 7B6 according to SEQ ID No. 21.

In embodiments, V_{L} and V_{H} are connected via a linker.

In embodiments, the tag-binding domain binding a human La epitope E5B9 comprises a heavy chain variable region (V_{H}) having complementary-determining regions (CDRs) with an amino acid sequence according to SEQ ID No. 22 (CDR-H1), an amino acid sequence according to SEQ ID No. 23 (CDR-H2), and an amino acid sequence according to SEQ ID No. 24 (CDR-H3), and a light chain variable region (V_{L}) having CDRs with an amino acid sequence according to SEQ ID No. 25 (CDR-L1), the amino acid sequence WAS (Trp-Ala-Ser) (CDR-L2), and an amino acid sequence according to SEQ ID No. 26 (CDR-L3).

In embodiments, the tag-binding domain is an antibody or an antigen-binding fragment comprising a V_{L} according to the following sequence:
DIVMTQSPDSLAVSLGERATI NCX₂₄SSQSLLNSRTX₃₅KNYLAWYQQKPGQPPKLLIYWASTR X₆₁SGVPDRFSGSGSGTDFTLTISSLQAEDVAVYYCKQSYNLX₁₀₁TFGGGTKVEIK (SEQ ID No. 27), wherein X₂₄, X₃₅, X₆₁ and X₁₀₁ are independently from each other selected from a proteinogenic alpha-amino acid residue; or an amino acid sequence having at least 90 % sequence identity, preferably at least 95 % sequence identity, more preferably at least 99 % sequence identity; to sequence SEQ ID No. 28 or SEQ ID No. 30.

In some embodiments, X₂₄ to X₁₀₁ are selected as follows:
X₂₄ is selected from polar and/or positive charged residues, such as Serine, Threonine, Asparagine, Glutamine, Histidine, Lysine and Arginine; preferably Lysine or Arginine;
X₃₅ is preferably selected from Lysine and Proline;
X₆₁ is selected from polar and charged residues, such as Asparagine, Aspartic Acid, Glutamine, Glutamic acid, Histidine, Lysine and Arginine, preferably Glutamic acid and Lysine;
X₁₀₁ is selected from hydrophobic residues, such as Isoleucine, Leucine, Valine, Alanine, Methionine, Phenylalanine, Proline and Tryptophan; preferably Leucine or Proline.

In embodiments, the tag-binding domain is an antibody or an antigen-binding fragment comprising a V_{H} with an amino acid sequence having at least 90 % sequence identity, preferably at least 95 % sequence identity, more preferably at least 99 % sequence identity; to sequence SEQ ID No. 29 or SEQ ID No. 31.

In embodiments, the tag-binding domain comprises a sequence having each at least 90 % sequence identity, preferably at least 95 % sequence identity, more preferably at least 99 % sequence identity; to the sequences according to SEQ ID No. 28 (V_{L}) and SEQ ID No. 29 (V_{H}).

In embodiments, the tag-binding domain is an anti-La 5B9 scFv according to SEQ ID No. 28 (V_{L}) and SEQ ID No. 29 (V_{H}).

In embodiments, the tag-binding domain comprises a sequence having each at least 90 % sequence identity, preferably at least 95 % sequence identity, more preferably at least 99 % sequence identity; to the sequences according to SEQ ID No. 30 (V_{L}) and SEQ ID No. 31 (V_{H}).

In embodiments, the tag-binding domain is an anti-La 7B6 scFv according to SEQ ID No. 30 (V_{L}) and SEQ ID No. 31 (V_{H}).

In embodiments, the tag-binding domain comprises a V_{L}-linker-V_{H} structure, wherein the V_{L} region of the tag-binding domain comprises a sequence with at least 95 % identity, preferably 99 % identity, with the sequence according to SEQ ID No. 28 and/or the V_{H} region of the tag-binding domain comprises a sequence with at least 95 % identity, preferably 99 % identity, with the sequence according to SEQ ID No. 29. As used herein, the term "V_{L}-linker-V_{H} structure" refers to a structure, wherein the C-terminus of the V_{L} region is connected with a linker, which is connected to the N-terminus of the V_{H} region.

In embodiments, the tag-binding domain is a Fab fragment binding a La epitope. In embodiments, the tag-binding domain is a Fab fragment binding La epitope 5B9 comprising an amino acid sequence according to SEQ ID No. 28 and SEQ ID No. 29.

In embodiments, the tag-binding domain or tag is present at the amino-terminal end of the polypeptide that comprises the switchable CAR. Advantageously, locating the tag-binding domain or the tag at the amino terminus permits unhampered access to the targeting module that is bound to the target cell. In embodiments, the RevCAR comprises a tag present at the amino-terminal end of the polypeptide.

As used herein, the term "extracellular hinge domain" refers to a peptide sequence between the transmembrane domain and the target cell-binding domain, tag-binding domain or tag, which protrudes from the surface of the cell for optimal binding of the CAR to its particular targeting cell or targeting module. Advantageously, the extracellular hinge domain enables different alignments of the CAR and thus the positioning of the CAR (effector cell) to the target cell, affecting the binding of the target cell by the CAR (effector cell). Despite the shortening of the extracellular domain, an increase in efficiency was observed. Further advantageously, the extracellular hinge domain enables efficient immune synapse formation.

Suitably, the extracellular hinge domain of the CAR polypeptide is linked to the transmembrane domain and the transmembrane domain is linked to the at least one intracellular signaling domain.

In embodiments, a CD19-binding domain or a CD20-binding domain of the chimeric antigen receptor polypeptide is linked to the extracellular hinge domain, preferably the CD19-binding domain of the chimeric antigen receptor polypeptide is linked to the extracellular hinge domain.

In embodiments, the CD20-binding domain of the chimeric antigen receptor polypeptide is linked to the CD19-binding domain.

In embodiments, the different domains of the CAR polypeptide are linked with each other in the order target cell-binding domain, extracellular hinge domain, transmembrane domain and at least one intracellular signaling domain. In embodiments, the different domains of the switchable CAR polypeptide are linked with each other in the order tag-binding domain or tag, extracellular hinge domain, transmembrane domain and at least one intracellular signaling domain.

In embodiments, the first and the second extracellular hinge domain are independently from each other selected from the group consisting of an extracellular hinge domain of CD4, CD8α, CD28, ICOS (CD278), IgG1, IgG2, IgG4 or mutants thereof. In embodiments, the first and the second extracellular hinge domain are independently from each other selected from the group consisting of an extracellular hinge domain of CD4, CD8α, preferably according to SEQ ID No. 32 or SEQ ID No. 33, CD28, preferably according to SEQ ID No. 34 or SEQ ID No. 35, ICOS (CD278), IgG1, preferably according to SEQ ID No. 37, IgG2, preferably according to SEQ ID No. 38, IgG4, preferably according to SEQ ID No. 36, or mutants thereof. In embodiments, the first and the second extracellular hinge domain are independently from each other selected from the group consisting of an extracellular hinge domain of CD8α according to SEQ ID No. 32 or SEQ ID No. 33, CD28 according to SEQ ID No. 34 or SEQ ID No. 35, IgG1 according to SEQ ID No. 37, IgG2 according to SEQ ID No. 38 or IgG4 according to SEQ ID No. 36 and mutants thereof. In embodiments, the first and the second extracellular hinge domain are independently from each other selected from the group consisting of an extracellular hinge domain of CD8α according to SEQ ID No. 32 or SEQ ID No. 33, CD28 according to SEQ ID No. 34 or SEQ ID No. 35, IgG1 according to SEQ ID No. 37, IgG2 according to SEQ ID No. 38 or IgG4 according to SEQ ID No. 36.

In embodiments, the first and/or the second extracellular hinge domain is in the range of 12 to 133 amino acids, preferably in the range of 12 to 62 amino acids, more preferably in the range of 12 to 38 amino acids.

As used herein, the term "mutants" refers to peptides or proteins having at least 90 % sequence identity to the named sequences, preferably at least 95 % sequence identity, more preferably at least 99 % sequence identity.

In embodiments, the extracellular hinge domain is the extracellular hinge domain of CD28 according to SEQ ID No. 34 or SEQ ID No. 35.

As used herein, the term "transmembrane domain" refers to a membrane-spanning protein domain or the part of the extracellular hinge and transmembrane domain, which anchors the CAR into the cell membrane of the cell. In embodiments, a transmembrane domain consists of at least one alpha-helix or a transmembrane beta barrel.

In embodiments, the first and the second transmembrane domain are independently from each other selected from the group consisting of a transmembrane domain of CD4, CD8α, CD28, ICOS (CD278), IgG1, IgG2, IgG4 or mutants thereof. In embodiments, the first and the second transmembrane domain are independently from each other selected from the group consisting of a transmembrane domain of CD4, preferably according to SEQ ID No. 44, CD8α, preferably according to SEQ ID No. 39 or SEQ ID No. 40, CD28, preferably according to SEQ ID No. 41 or SEQ ID No. 42, ICOS (CD278), preferably according to SEQ ID No. 43, IgG1, IgG2, IgG4 or mutants thereof. In embodiments, the first and the second transmembrane domain are independently from each other selected from the group consisting of a transmembrane domain of CD4 according to SEQ ID No. 44, CD8α according to SEQ ID No. 39 or SEQ ID No. 40, CD28 according to SEQ ID No. 41 or SEQ ID No. 42, ICOS (CD278) according to SEQ ID No. 43 and mutants thereof. In embodiments, the first and the second transmembrane domain are independently from each other selected from the group consisting of a transmembrane domain of CD4 according to SEQ ID No. 44, CD8α according to SEQ ID No. 39 or SEQ ID No. 40, CD28 according to SEQ ID No. 41 or SEQ ID No. 42, ICOS (CD278) according to SEQ ID No. 43.

In embodiments, the transmembrane domain is the transmembrane domain of CD28 according to SEQ ID No. 41 or SEQ ID No. 42.

In embodiments, combinations of the extracellular hinge and transmembrane domain are CD8α extracellular hinge and transmembrane domain, CD28 extracellular hinge and transmembrane domain, a CD28 extracellular hinge domain combined with a CD8α transmembrane domain, an IgG4 extracellular hinge domain combined with a CD8α transmembrane domain or an IgG4 extracellular hinge domain combined with a CD28 transmembrane domain.

In embodiments, the mutant of the extracellular hinge domain and/or the transmembrane domain comprises one- or two-point mutations. In embodiments, the mutants are truncated variants of the extracellular hinge domain and/or the transmembrane domain, in particular domains having a chain length of at least 90 % and a sequence identity of 100 %, most preferably a chain length of at least 95 % and a sequence identity of 100 %, resulting from a mutation in the nucleotide sequence coding for the domain.

In embodiments, the first and the second extracellular hinge domains are the same or different In embodiments, the first and the second transmembrane domains are the same or different.

In embodiments, the first and the second extracellular hinge domains and the first and the second transmembrane domains are different. Suitably, using different extracellular hinge domains and different transmembrane domains enables only an ideal signaling when at least one target and one targeting module is present.

In preferred embodiments, the first and the second extracellular hinge domains and the first and the second transmembrane domains are the same. Advantageously, using the same extracellular hinge domains and the same transmembrane domains enables an ideal signaling when either target or targeting module is present as the domains are able to dimerize.

As used herein, the term "intracellular signaling domain" refers to a peptide sequence which transmits a signal into the cell by cross-linkage of the cell expressing the CAR (effector cell) to a human cell surface protein or protein complex (target cell). Cross-linkage between effector and target cell is mediated by the target-binding domain or by the targeting module.

In embodiments, the at least one first and the at least one second intracellular signaling domain are independently from each other selected from the group consisting of a cytoplasmic domain of CD3, CD27, CD28, OX40 (CD134), 4-1BB (CD137), ICOS (CD278), DAP10, DAP12, PD-1, CTLA-4, IL-2 receptor, IL-7 receptor, IL-15 receptor or IL-21 receptor and mutants thereof. In embodiments, the at least one intracellular signaling domain is selected from the group consisting of a cytoplasmic domain of CD3, preferably according to SEQ ID No. 45, CD27, preferably according to SEQ ID No. 46, CD28, preferably according to SEQ ID No. 47 or SEQ ID No. 48, CD134 (OX40), preferably according to SEQ ID No. 49, CD137 (4-1BB), preferably according to SEQ ID No. 50 or SEQ ID No. 51, CD278 (ICOS), preferably according to SEQ ID No. 52, DAP10, preferably according to SEQ ID No. 53, DAP12, preferably according to SEQ ID No. 54, PD-1, preferably according to SEQ ID No. 55, CTLA-4, preferably according to SEQ ID No. 56, IL-2 receptor, preferably CD122 (interleukin-2 receptor β) or CD132 (interleukin-2 receptor γ); IL-7 receptor, preferably CD127 (interleukin-7 receptor α); IL-15 receptor or CD360 (interleukin-21 receptor), and mutants thereof. In embodiments, the at least one intracellular signaling domain is selected from the group consisting of a cytoplasmic domain of CD3 according to SEQ ID No. 45, CD27 according to SEQ ID No. 46, CD28 according to SEQ ID No. 47 or SEQ ID No. 48, CD134 (OX40) according to SEQ ID No. 49, CD137 (4-1BB) according to SEQ ID No. 50 or SEQ ID No. 51, CD278 (ICOS) according to SEQ ID No. 52, DAP10 according to SEQ ID No. 53, DAP12 according to SEQ ID No. 54, PD-1 according to SEQ ID No. 55, CTLA-4 according to SEQ ID No. 56 and mutants thereof.

As used herein, the term "mutants" refers to proteins having at least 90 % sequence identity to the cytoplasmic domains, preferably at least 95 % sequence identity, more preferably at least 99 % sequence identity. Advantageously, the mutant transmits a signal into the cell by cross-linkage of the cell expressing the CAR (effector cell) to a human cell surface protein or protein complex (target cell) in the same way as the named intracellular signaling domains.

In embodiments, mutants are truncated versions. As used herein, the term "truncated versions" refers to shortened proteins having at least 90 % sequence identity to the cytoplasmic domains, preferably at least 95 % sequence identity, more preferably having a chain length of at least 90 % and a sequence identity of 100 %, most preferably a chain length of at least 95 % and a sequence identity of 100 %. Advantageously, the truncated version has an activity of at least 80%, preferably of at least 90 %, more preferably of at least 95 %; of the named cytoplasmic domains.

In embodiments, the at least one intracellular signaling domain is selected from the group consisting of a cytoplasmic domain of CD3 according to SEQ ID No. 45, CD27 according to SEQ ID No. 46, CD28 according to SEQ ID No. 47 or SEQ ID No. 48, CD134 (OX40) according to SEQ ID No. 49, CD137 (4-1BB) according to SEQ ID No. 50 or SEQ ID No. 51, CD278 (ICOS) according to SEQ ID No. 52, DAP10 according to SEQ ID No. 53, DAP12 according to SEQ ID No. 54, PD-1 according to SEQ ID No. 55 and CTLA-4 according to SEQ ID No. 56.

In embodiments, the CAR polypeptide comprises at least two intracellular signaling domains independently selected from a cytoplasmic region of a cytoplasmic domain of CD3, preferably according to SEQ ID No. 45, CD27, preferably according to SEQ ID No. 46, CD28, preferably according to SEQ ID No. 47 or SEQ ID No. 48, CD134 (OX40), preferably according to SEQ ID No. 49, CD137 (4-1BB), preferably according to SEQ ID No. 50 or SEQ ID No. 51, CD278 (ICOS), preferably according to SEQ ID No. 52, DAP10, preferably according to SEQ ID No. 53, DAP12, preferably according to SEQ ID No. 54, PD-1, preferably according to SEQ ID No. 55, CTLA-4, preferably according to SEQ ID No. 56, IL-2 receptor, preferably CD122 (interleukin-2 receptor β) or CD132 (interleukin-2 receptor γ); IL-7 receptor, preferably CD127 (interleukin-7 receptor α); IL-15 receptor or CD360 (interleukin-21 receptor), and mutants thereof.

Suitably, the first intracellular signaling domain and the second intracellular signaling domain of the CAR polypeptide are different.

In embodiments, the switchable CAR polypeptide comprises at least two intracellular signaling domains independently selected from a cytoplasmic domain of CD3, preferably according to SEQ ID No. 45, CD27, preferably according to SEQ ID No. 46, CD28, preferably according to SEQ ID No. 47 or SEQ ID No. 48, CD134 (OX40), preferably according to SEQ ID No. 49, CD137 (4-1BB), preferably according to SEQ ID No. 50 or SEQ ID No. 51, CD278 (ICOS), preferably according to SEQ ID No. 52, DAP10, preferably according to SEQ ID No. 53, DAP12, preferably according to SEQ ID No. 54, PD-1, preferably according to SEQ ID No. 55, CTLA-4, preferably according to SEQ ID No. 56, IL-2 receptor, preferably CD122 (interleukin-2 receptor β) or CD132 (interleukin-2 receptor γ); IL-7 receptor, preferably CD127 (interleukin-7 receptor α); IL-15 receptor or CD360 (interleukin-21 receptor), and mutants thereof.

Suitably, the first intracellular signaling domain and the second intracellular signaling domain of the switchable CAR polypeptide are different.

In embodiments, the at least one first and the at least one second intracellular signaling domains are the same or different. In embodiments, the CAR polypeptide comprises at least two intracellular signaling domains and the switchable CAR polypeptide comprises at least two intracellular signaling domains, wherein the at least two intracellular signaling domains of the CAR polypeptide and the switchable CAR polypeptide are the same or different.

In embodiments, the CAR polypeptide comprises at least two intracellular signaling domains and the switchable CAR polypeptide comprises at least two intracellular signaling domains, wherein the at least one intracellular signaling domain of the CAR polypeptide and the switchable CAR polypeptide are the same and at least one intracellular signaling domain of the CAR polypeptide and the switchable CAR polypeptide are different.

In embodiments, the first intracellular signaling domain of the CAR polypeptide comprises a cytoplasmic region of a CD3ζ according to SEQ ID No. 45 and the second intracellular signaling domain of the CAR polypeptide comprises a cytoplasmic region of a CD137 (4-1BB) according to SEQ ID No. 50 or SEQ ID No. 51.

In embodiments, the first intracellular signaling domain of the switchable CAR polypeptide comprises a cytoplasmic region of a CD3ζ according to SEQ ID No. 45 and the second intracellular signaling domain of the switchable CAR polypeptide comprises a cytoplasmic region of a CD28 according to SEQ ID No. 47 or SEQ ID No. 48.

In embodiments, the tag is a peptide epitope tag, preferably a myc-tag, a His-tag, a linear peptide sequence from yeast transcription factor GCN4, a leucine zipper sequence or a peptide sequence from a human nuclear protein, preferably from the human La protein.

In embodiments, the tag-binding domain is an antibody, antigen-binding fragment, a protein or a peptide binding to a myc-tag, a His-tag, a peptide sequence from yeast transcription factor GCN4, a leucine zipper sequence or a peptide sequence from a human protein.

In further embodiments, the CAR polypeptide and/or the switchable CAR polypeptide comprise a further domain, wherein the further domain is a short peptide linker in the extracellular portion of the CAR that may serve to detect the CAR on the cell surface or stimulate the CAR-T cell. Advantageously, the CAR engrafted cells with the further domain can be specifically stimulated to proliferate preferentially and persist longer compared to non-engrafted cells either *in vitro* or *in vivo.* Further advantageously, the further domain may also be used to purify CAR engrafted cells from mixed cell populations or to dampen CAR engrafted cell-mediated immune response and to eliminate CAR engrafted cells *in vivo.*

In embodiments, the further domain is a linear epitope, which can specifically be bound by a monoclonal antibody (mab). In some embodiments, the further domain comprises E7B6 according to SEQ ID No. 20 or SEQ ID No. 21.

In embodiments, the further domain is located in between the target-binding domain or the tag-binding domain or tag and the extracellular hinge domain or is an integral part of the extracellular hinge domain.

In embodiments, the at least one CAR polypeptide and/or the at least one switchable CAR polypeptide comprise a signal peptide selected from human CD8α, preferably according to SEQ ID No. 61, CSF2Ra, preferably according to SEQ ID No. 60, CD3ζ, preferably according to SEQ ID No. 59, IL-2, preferably according to SEQ ID No. 58, lysozyme C, a heavy chain of an antibody, a light chain of an antibody or a part of a light chain of an antibody. In embodiments, the at least one CAR polypeptide and/or the at least one switchable CAR polypeptide comprise a signal peptide selected from the group comprising SEQ ID No. 57 to SEQ ID No. 61. In embodiments, the at least one CAR polypeptide comprises a signal peptide selected from human CD8α according to SEQ ID No. 61, CSF2Ra according to SEQ ID No. 60, CD3ζ according to SEQ ID No. 59, IL-2 according to SEQ ID No. 58, and Igκ according to SEQ ID No. 57.

As used herein, the term "signal peptide" (also leader peptide) refers to a short amino acid sequence at the N-terminus of proteins meant for secretion or membrane localization. Advantageously, the signal peptide effects the transport of the CAR on the cell surface of an effector cell.

Suitably, the signal peptide is located at the N-terminus of the CAR sequence ahead of the target-binding domain or tag-binding domain or tag, the extracellular hinge and transmembrane domain and the intracellular signaling domain.

In embodiments, the CAR polypeptide and/or the at least one switchable CAR polypeptide comprise a signal peptide of a human immunoglobulin with an amino acid sequence in the range of 16 to 30 amino acids. The term "immunoglobulin" refers to Y-shaped protein consisting of two identical heavy chains and two identical light chains connected by disulfide bonds used by the immune system to identify and neutralize foreign objects such as pathogenic bacteria and viruses, called antigens. Advantageously, using signal peptides influences the CAR expression levels on the surface of an effector cell and thus, the efficiency.

In embodiments, the at least one CAR polypeptide and/or the at least one switchable CAR polypeptide comprise a sequence with a cleavage site, preferably according to SEQ ID No. 62 or SEQ ID No. 63.

In embodiments, the at least one CAR polypeptide comprises one of the sequences selected from SEQ ID No. 65 to SEQ ID No. 67 or a sequence with an identity of at least 95 %, preferably at least 99 %, with one of the sequences selected from SEQ ID No. 65 to SEQ ID No. 67.

In embodiments, the at least one switchable CAR polypeptide comprises one of the sequences selected from SEQ ID No. 74 to SEQ ID No. 79 or a sequence with an identity of at least 95 %, preferably at least 99 %, with one of the sequences selected from SEQ ID No. 74 to SEQ ID No. 79.

In embodiments, the at least one CAR polypeptide and/or the at least one switchable CAR polypeptide comprise one of the sequences selected from SEQ ID No. 86 to SEQ ID No. 92 or a sequence with an identity of at least 95 %, preferably at least 99 %, with one of the sequences selected from SEQ ID No. 86 to SEQ ID No. 92. In embodiments, the at least one CAR polypeptide and/or the at least one switchable CAR polypeptide comprise SEQ ID No. 86 or a sequence with an identity of at least 95 %, preferably at least 99 %, with SEQ ID No. 86.

In embodiments, the engineered cell comprises at least one further CAR polypeptide comprising a further target cell-binding domain, an extracellular hinge domain, a transmembrane domain and at least one intracellular signaling domain.

In embodiments, the engineered cell comprises at least one further switchable CAR polypeptide comprising a further tag-binding domain or tag, an extracellular hinge domain, a transmembrane domain and at least one intracellular signaling domain.

In embodiments, the cell comprises an exogenous nucleotide sequence encoding the CAR polypeptide that is expressed on the surface of the cell.

In embodiments, the cell comprises an exogenous nucleotide sequence encoding the switchable CAR polypeptide that is expressed on the surface of the cell.

In embodiments, the cell is selected from immune cells, preferably with cytolytic, phagocytic or immunosuppressive activity, such as T cells, Natural Killer (NK) cells and macrophages. In embodiments, the cell is selected from T cells, including alpha/beta and gamma/delta T cells or subpopulations of T cells like stem-cell memory T cells, central memory T cells, regulatory T cells, cytotoxic T cells or NK cells.

In embodiments, the cell is an engineered human cell comprising at least one genetic modification.

In embodiments, the cell is an engineered allogeneic immune cell. In embodiments, the engineered allogeneic immune cell is obtained by at least one genetic modification of a donor cell.

In embodiments, the cell comprises a genetic modification in a T cell receptor alpha chain (TRAC) gene that reduces or eliminates surface expression of endogenous T cell receptor alpha chain relative to an unmodified T cell, a genetic modification in an HLA-A gene that reduces or eliminates surface expression of endogenous HLA-A relative to an unmodified T cell and a genetic modification in a CIITA gene that reduces or eliminates surface expression of endogenous HLA class II relative to an unmodified T cell. Advantageously, this approach results in a reduction of the rejection by the recipient subject's immune cells, in particular decrease the chance of GvHD for allogeneic T cells

In embodiments, the cell comprises a genetic modification in a CD52 (CAMPATH-1 antigen) gene that reduces or eliminates surface expression of endogenous CD52 to an unmodified T cell and a genetic modification in a TCR (T cell receptor) gene that reduces or eliminates surface expression of endogenous TCR relative to an unmodified T cell. Suitably, alemtuzumab is administered to achieve long-term lymphodepletion.

In embodiments, the cell comprises a genetic modification in a TCR gene that reduces or eliminates surface expression of endogenous TCR relative to an unmodified T cell.

In embodiments, the cell comprises a genetic modification in a TCR gene that reduces or eliminates surface expression of endogenous TCR relative to an unmodified T cell, a genetic modification in MHC class I gene that reduces or eliminates surface expression of endogenous MHC class I relative to an unmodified T cell and a genetic modification in MHC class II gene that reduces or eliminates surface expression of endogenous MHC class I relative to an unmodified T cell. Advantageously, this approach results in reduction of alloreactivity and immunogenicity.

In embodiments, the genetic modification in MHC class I gene is a genetic modification in B2M (Beta-2 microglobulin) gene.

In embodiments, the cell comprises a genetic modification in a TCR gene that reduces or eliminates surface expression of endogenous TCR relative to an unmodified T cell, a genetic modification in B2M gene that reduces or eliminates surface expression of endogenous MHC class I relative to an unmodified T cell and HLA-E overexpression for NK cell evasion.

In embodiments, the cell comprises a genetic modification in a TCR gene that reduces or eliminates surface expression of endogenous TCR relative to an unmodified T cell, a genetic modification in B2M gene that reduces or eliminates surface expression of endogenous MHC class I relative to an unmodified T cell and HLA-G overexpression for NK cell evasion.

In embodiments, the cell comprises a genetic modification in a TCR gene that reduces or eliminates surface expression of endogenous TCR relative to an unmodified T cell and CD47 overexpression. Advantageously, this approach results in the inhibition of phagocytosis and enhancement of persistence.

In embodiments, the cell comprises a genetic modification in a TCR gene that reduces or eliminates surface expression of endogenous TCR relative to an unmodified T cell and an overexpression of at least one inhibitory receptor (e.g., PD-L1). Advantageously, this approach results in the reduction of recognition by host immune cells.

In embodiments, the engineered cell according to the invention is used in the treatment of cancer, infectious disease or autoimmune disease.

The term "autoimmune disorder" refers to an abnormal immune response of the body against substances and tissues normally present in the body (autoimmunity).

In embodiments, the engineered cell according to the invention is used in the treatment of cancer. In embodiments, the engineered cell according to the invention is used in the treatment of tumors of the hematopoietic and lymphoid tissues. In embodiments, the engineered cell according to the invention is used in the treatment of blood cancer. In embodiments, the engineered cell according to the invention is used in the treatment of b cell lymphomas.

In embodiments, the engineered cell according to the invention is used for the manufacture of a medicament for the treatment of cancer, infectious disease or autoimmune disease.

In embodiments, the engineered cell according to the invention is used in a method of treating cancer, infectious disease or autoimmune disease in a subject in need thereof, wherein the engineered cell according to the invention is administered to a subject in need thereof.

In embodiments, the method of treating cancer, infectious disease or autoimmune disease comprises the administration of the engineered cell according to the invention or a pharmaceutical composition to a subject in need thereof, preferably a mammal, more preferably a human, having cancer, an infectious or an autoimmune disease. For therapeutic applications, a sterile pharmaceutical composition comprising a pharmacologically effective quantity of the engineered cell according to the invention, is administered to a subject in order to treat the aforementioned illnesses.

In embodiments, the pharmaceutical composition is administered parenterally, particularly intravenously.

In embodiments, the pharmaceutical composition comprises the engineered cell according to the invention in a dosage quantity in the range of 5·10⁷ to 1·10⁹ cells within day 0 to day 5 of the treatment. In embodiments, the pharmaceutical composition comprises the engineered cell according to the invention in a dosage quantity in the range of 5·10⁷ to 5·10⁸ cells within day 0 to day 5 of the treatment.

Another aspect of the invention is a pharmaceutical composition comprising the engineered cell according to the invention and a pharmaceutically acceptable thinner or carrier.

In embodiments, the pharmaceutical composition is present in a form suitable for intravenous administration. In embodiments, the pharmaceutical composition is a solution, emulsion or suspension.

In preferred embodiments, the pharmaceutical composition is an injectable buffered solution comprising the engineered cell according to the invention with a concentration in the range of 1·10⁵ to 1·10⁸ per mL.

The pharmaceutical composition comprises a pharmaceutically acceptable thinner (dilution agent) or carrier. In embodiments, the carrier is selected from water, an aqueous buffer solution, 0.9 % saline solution, 5 % glucose, 5 % xylitol, 0.3 % glycine solution, ringer solutions or amino acid solutions. In embodiments, the aqueous buffer solution is selected from an aqueous histidine, sodium succinate, sodium citrate, sodium acetate, sodium phosphate or potassium phosphate-buffered solution with a pH value in the range of pH 5.0 to pH 7.0. In embodiments, the aqueous buffer solution has a buffer concentration in the range of 1 mmol/l (mM) to 500 mM. In embodiments, the aqueous buffer solution has a buffer concentration in the range of 5 mM to 20 mM. In embodiments, the aqueous buffer solution has a buffer concentration in the range of 5 mM to 10 mM.

In embodiments, the carrier comprises sodium chloride. In embodiments, the carrier comprises sodium chloride with a concentration in the range of 1 mM to 300 mM. In embodiments, the carrier comprises sodium chloride with a concentration in the range of 140 mM to 160 mM.

In embodiments, the pharmaceutical composition comprises a stabilizer. In embodiments, the pharmaceutical composition comprises a stabilizer with a concentration in the range of 1 mM to 900 mM. In embodiments, the pharmaceutical composition comprises a stabilizer with a concentration in the range of 50 mM and 600 mM. In embodiments, the stabilizer is sucrose, trehalose or L-methionine.

In embodiments, the pharmaceutical composition comprises pharmaceutically acceptable excipients. As used herein, the term "pharmaceutically acceptable excipients" refers to compounds, which provide approximately physiological conditions and/or increase the stability, such as agents for adjusting the pH value and buffering agents, agents for adjusting the toxicity and the like. In embodiments, pharmaceutically acceptable excipients are selected from sodium acetate, sodium chloride, potassium chloride, calcium chloride, sodium lactate and polysorbate-80. In embodiments, the pharmaceutically acceptable excipient is in the range of 0.0001 % (w/v) to 1 % (w/v). In embodiments, the pharmaceutically acceptable excipient is in the range of 0.001 % (w/v) to 0.1 % (w/v).

In embodiments, the pharmaceutical composition is sterile. In embodiments, the pharmaceutical composition is sterilized by conventional well-known techniques including, but not limited to, sterile filtration.

In embodiments, the pharmaceutical composition is used for administration to a subject.

In embodiments, the pharmaceutical composition is lyophilized prior to storage or stored as solution at ambient temperature or below, including, but not limited to, frozen storage.

In embodiments, the pharmaceutical composition is reconstituted and/or diluted in an infusion and stabilizer solution prior to administration to a subject. The solutions used for reconstitution, infusion and/or stabilization may contain any of the components mentioned for the pharmaceutical composition or similar components.

In embodiments, the pharmaceutical composition is used in a method for treatment of cancer, infectious disease or autoimmune disease.

A further aspect of the invention is a kit comprising the at least one engineered cell according to the invention and/or
i) at least one first nucleic acid or vector comprising a nucleotide sequence encoding the at least one CAR polypeptide comprising:
   - at least one target cell-binding domain,
   - a first extracellular hinge domain,
   - a first transmembrane domain, and
   - at least one first intracellular signaling domain, and
ii) at least one second nucleic acid or vector comprising a nucleotide sequence encoding the at least one switchable CAR polypeptide comprising:
   - a tag-binding domain or a tag,
   - a second extracellular hinge domain,
   - a second transmembrane domain, and
   - at least one second intracellular signaling domain and/or
at least one nucleic acid or vector comprising a nucleotide sequence encoding the at least one CAR polypeptide and the at least one switchable CAR polypeptide.

In embodiments, the at least one first nucleic acid or vector comprises at least one nucleic acid sequence encoding one of the sequences according to SEQ ID No. 65 to SEQ ID No. 67.

In embodiments, the at least one first nucleic acid or vector comprises at least one nucleic acid sequence according to one of the sequences of SEQ ID No. 68 to SEQ ID No. 73.

In embodiments, the at least one second nucleic acid or vector comprises at least one nucleic acid sequence encoding one of the sequences according to SEQ ID No. 74 to SEQ ID No. 79.

In embodiments, the at least one second nucleic acid or vector comprises at least one nucleic acid sequence according to one of the sequences of SEQ ID No. 80 to SEQ ID No. 85.

In embodiments, the at least one nucleic acid or vector comprises at least one nucleic acid sequence encoding one of the sequences according to SEQ ID No. 86 to SEQ ID No. 92.

In embodiments, the at least one nucleic acid or vector comprises at least one nucleic acid sequence according to one of the sequences of SEQ ID No. 93 to SEQ ID No. 99.

Suitably, the nucleic acid and/or vector are isolated.

In embodiments, the at least one first and/or the at least one second nucleic acid are cDNA. As used herein, the term "cDNA" (complementary DNA) refers to double-stranded DNA synthesized from a single-stranded RNA, e.g. mRNA, in a reaction catalyzed by the enzyme reverse transcriptase. In embodiments, cDNA is of synthetic origin. In further embodiments, cDNA is derived from mRNA, therefore containing only exons but no introns, as opposed to genomic DNA.

In embodiments, the at least one first and/or the at least one second vector are selected from the group consisting of a DNA vector, an RNA vector, a plasmid, a lentiviral vector, retroviral vector, adenoviral vector and an adeno-associated viral vector.

In embodiments, the at least one first and/or the at least one second vector further comprise a promoter, wherein the promoter is selected from the group comprising an EF-1 promoter, a CMV IE gene promoter, an EF-1α promoter, a ubiquitin C promoter, or a phosphoglycerate kinase (PGK) promoter. In embodiments, the nucleic acid or vector comprises at least one promoter according to SEQ ID No. 64.

In embodiments, the vector or cell further comprises an inducible expression system. In some embodiments, the inducible expression system is based on a prokaryotic operon, including, but not limited to, the lac operon, transposon Tn₁₀ or tetracycline operon. In other embodiments, the inducible expression system is based on components of a eukaryotic signaling pathway, including, but not limited to, expression systems based on a steroid receptor, an estrogen receptor, progesterone or metallothionein.

In embodiments, the inducible expression system induces the transcription of the nucleotide sequence encoding the at least one CAR polypeptide and/or the nucleotide sequence encoding the at least one switchable CAR polypeptide.

In embodiments, the kit further comprises at least one targeting module comprising at least one further target cell-binding domain capable of binding a target antigen and a tag or tag-binding domain and/or at least one nucleic acid, vector and/or cell encoding a targeting module,
wherein the further target cell-binding domain is different from the target cell-binding domain of the CAR polypeptide,
wherein the tag-binding domain of the targeting module binds to the tag of the switchable CAR or the tag of the targeting module binds to the tag-binding domain of the switchable CAR.

In embodiments, the targeting module is isolated. In embodiments, the targeting module is expressed as a recombinant protein. In further embodiments, the targeting module is chemically synthesized.

In embodiments, the targeting module is in monomeric, dimeric or polymeric form, preferably in monomeric form.

In embodiments, the targeting module comprises a tag. Suitably, the targeting module comprising a tag is specific for the tag-binding domain of a UniCAR.

In embodiments, the targeting module comprises a tag-binding domain. Suitably, the targeting module comprising a tag-binding domain is specific for the tag of a RevCAR.

In embodiments, the targeting module comprises a further domain selected from the group comprising co-stimulatory ligands, radionuclides, cell death-inducing chemical compounds and half-life increasing domains, preferably IgG1 Fc, IgG2 Fc, IgG3 Fc, IgG4 Fc, HSA, FcRn-binding peptides or mutants thereof. As used herein, the term "mutants" refers to proteins having at least 90 % sequence identity to the half-life increasing domain, preferably at least 95 % sequence identity. Advantageously, the mutant is capable of having one or more activities of the named peptides or proteins; in particular, the mutant increases the half-life like the half-life increasing domain.

In embodiments, the target cell-binding domain of the CAR and the further target cell-binding domain of the targeting module are independently from each other selected from an antibody, antigen-binding fragment, a protein, a peptide or a low molecular weight organic ligand that binds to a surface antigen selected from the group consisting of CD2, CD3, CD4, CD5, CD7, CD8, CD10, CD15, CD19, CD20, CD22, CD23, CD25, CD30, CD33, CD38, CD44, CD44v6, CD52, CD66a, CD66b, CD66c, CD66d, CD66e, CD66f, CD79a, CD79b, CD90, CD99, CD123, CD133, CD135, CD150, CD181, CD182, CD184, CD223, CD229, CD269, CD273, CD274, CD276, CD279, CD319, CD366, CD371, a cytokine receptor, CXCR4, c-Met, mesothelin, a member of the epidermal growth factor receptor family or a mutant thereof, a member of the tumor necrosis factor receptor superfamily, a claudin, an ephrin, an ephrin receptor, a fucosyl transferase, a prostate specific antigen, an embryonic antigen, a member of the vascular endothelia growth factor family, epithelial cell adhesion molecule, alpha-fetoprotein, a member of the intercellular adhesion molecule family, a C-type lectin, an integrin, a member of the mucin protein family, a follicle-stimulating hormone receptor, a high molecular weight-melanoma associated antigen, a folate binding protein, a folate receptor, a somatostatin receptor, a ligand of the NKG2D receptor, a member of the epithelia glycoprotein family, a disialoganglioside, a glypican, a G protein-coupled receptor, a human papillomavirus protein, cancer/testis antigen, fibroblast activation protein, a member of the carbonic anhydrase family, a member of the carbohydrate antigen family, a Notch ligand, melanoma-associated chondroitin sulfate proteoglycan, glycoprotein A33, guanylate cyclase 2C and tumor-specific glycan.

In embodiments, the target cell-binding domain of the CAR and the further target cell-binding domain of the targeting module are independently from each other selected from an antibody, antigen-binding fragment, a protein, a peptide or a low molecular weight organic ligand that binds to a surface antigen selected from the group consisting of CD2, CD3, CD4, CD5, CD7, CD8, CD10, CD15, CD19, CD20, CD22, CD23, CD25, CD30, CD33, CD38, CD44, CD44v6, CD52, CD66a, CD66b, CD66c, CD66d, CD66e, CD66f, CD79a, CD79b, CD90, CD99, CD123, CD133, CD135, CD150, CD181, CD182, CD184, CD223, CD229, CD269, CD273, CD274, CD276, CD279, CD319, CD366, CD371, a cytokine receptor, CXCR4, c-Met, mesothelin, a member of the epidermal growth factor receptor family, a member of the tumor necrosis factor receptor superfamily, a claudin, an ephrin, an ephrin receptor, a fucosyl transferase, a prostate specific antigen, an embryonic antigen, a member of the vascular endothelia growth factor family, epithelial cell adhesion molecule, alpha-fetoprotein, a member of the intercellular adhesion molecule family, a C-type lectin, an integrin, a member of the mucin protein family, a follicle-stimulating hormone receptor, a high molecular weight-melanoma associated antigen, a folate binding protein, a folate receptor, a somatostatin receptor, a ligand of the NKG2D receptor, a member of the epithelia glycoprotein family, a disialoganglioside, a glypican, a G protein-coupled receptor, a human papillomavirus protein, cancer/testis antigen, fibroblast activation protein, a member of the carbonic anhydrase family, a member of the carbohydrate antigen family, a Notch ligand, melanoma-associated chondroitin sulfate proteoglycan, glycoprotein A33, guanylate cyclase 2C and tumor-specific glycan.

In embodiments, the target cell-binding domain of the CAR and the further target cell-binding domain of the targeting module are independently from each other selected from an antibody, antigen-binding fragment, a protein, a peptide or a low molecular weight organic ligand that binds to a surface antigen selected from the group consisting of CD2, CD3, CD4, CD5, CD7, CD8, CD10, CD15, CD19, CD20, CD22, CD23, CD25, CD30, CD33, CD38, CD44, CD44v6, CD52, CD66a, CD66b, CD66c, CD66d, CD66e, CD66f, CD79a, CD79b, CD90, CD99, CD123, CD133, CD135, CD150, CD181, CD182, CD184, CD223, CD229, CD269, CD273, CD274, CD276, CD279, CD319, CD366, CD371, CXCR4, c-Met, mesothelin, a claudin, an ephrin, an ephrin receptor, a fucosyl transferase, a prostate specific antigen, an embryonic antigen, epithelial cell adhesion molecule, alpha-fetoprotein, a C-type lectin, a follicle-stimulating hormone receptor, a high molecular weight-melanoma associated antigen, a folate binding protein, a folate receptor, a somatostatin receptor, a ligand of the NKG2D receptor, a disialoganglioside, a glypican, a G protein-coupled receptor, a human papillomavirus protein, cancer/testis antigen, fibroblast activation protein, a Notch ligand, melanoma-associated chondroitin sulfate proteoglycan, glycoprotein A33, and guanylate cyclase 2C.

In embodiments, the at least one target cell-binding domain of the CAR and the further target cell-binding domain of the targeting module are independently from each other selected from an antibody, antigen-binding fragment, a protein, a peptide or a low molecular weight organic ligand that binds to a surface antigen selected from the group consisting of an antibody fragment that binds to BCMA (B-cell maturation antigen), CD19, CD20, CD38, CD123, IL13Rα2, HER-2, PD-L1 and/or PD-L2.

In embodiments, the length of the targeting module is in the range of 200 to 1600 amino acids, preferably 600 to 1600 amino acids.

In embodiments, the targeting module comprises the amino acid sequence according to SEQ ID No. 100 to SEQ ID No. 102.

In embodiments, the kit comprises one to three targeting modules.

In embodiments, the first and/or second vector are independently from each other selected from the group consisting of a DNA vector, an RNA vector, a plasmid, a lentiviral vector, retroviral vector, adenoviral vector and an adeno-associated viral vector.

In embodiments, the engineered cell and/or the targeting module are in the form of a pharmaceutical composition.

In embodiments, the kit comprises at least one engineered cell and at least one targeting module, for use in the treatment of cancer, infectious disease or autoimmune disease.

In embodiments, the engineered cell according to the invention, the pharmaceutical composition according to the invention or the kit according to the invention is used for preparing a medication for therapeutic and/or diagnostic use in case of cancer, an infection or an autoimmune disease.

In embodiments, the targeting module is administered in combination with the engineered cell according to the invention. As used herein, the term "administered in combination" refers to a treatment, wherein the targeting module is administered prior to, simultaneously with and/or after the administration of the cell comprising a nucleotide sequence encoding a switchable chimeric antigen receptor (CAR).

In embodiments, the targeting module is administered on its own, preferably one hour to 2 days, more preferably 4 to 24 hours, prior to the administration of the engineered cell according to the invention. Advantageously, the administration of the targeting module prior to the administration of the engineered cell stimulates the switchable CAR and increases the expansion of the CAR carrying effector cells and their accumulation at the target site.

In embodiments, the targeting module is administered simultaneously with the engineered cell.

In embodiments, the targeting module is administered until, preferably in the range of 3 days to 30 days, after the administration of the engineered cell. In embodiments, additional such doses of the targeting module may be administered following resting periods to reactivate the CAR-carrying effector cells.

For the realization of the invention, it is also convenient to combine the above-described embodiments, embodiments and features according to the invention.

The following detailed description of exemplary embodiments of the invention is presented to enable any person skilled in the art to make and use the disclosed subject matter in the context of one or more implementations. Various modifications to the disclosed implementations will be readily apparent to those skilled in the art, and the general principles defined herein may be applied to other implementations and applications without departing from scope of the disclosure. Therefore, this disclosure is not limited to the implementations described or illustrated but is to be given the widest scope consistent with the principles and features disclosed herein.

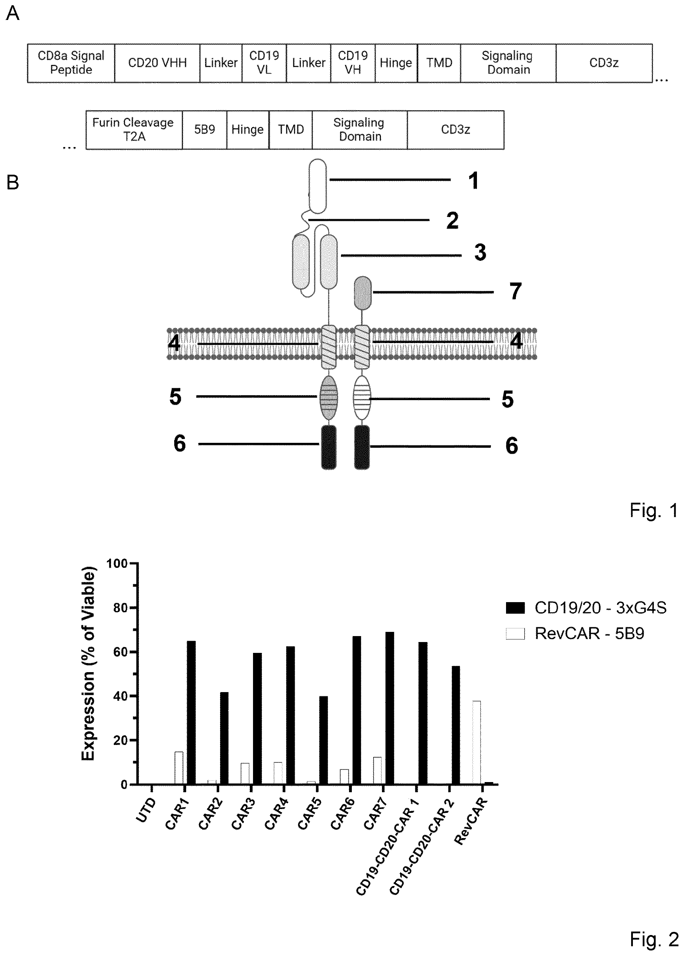

- **Fig. 1**: shows a schematic representation of an engineered cell according to the invention comprising a CD19 and CD20 dual targeting chimeric antigen receptor (CAR) and a switchable CAR. **A**) Linear diagram of an example of a CD19 and CD20 dual targeting CAR and switchable CAR according to the invention with CD8a signal peptide, CD20-targeting V_{H}H, linker, CD19-targeting scFv, hinge, transmembrane domain (TMD), co-stimulatory domain and CD3z domain. The switchable CAR is expressed inframe with the CAR using a furin cleavage site and T2A element. The switchable CAR is comprised of an IgGK signal peptide, 5B9 epitope tag, hinge, transmembrane domain and CD3z domain. **B**) scheme of an engineered cell according to the invention comprising a CD19 and CD20 dual targeting CAR and a switchable CAR. The carboxy-terminal CD20-targeting V_{H}H **1** is linked to the CD19-targeting scFv **3**. The targeting domains are connected to an extracellular hinge and transmembrane domain **4**, intracellular domains, in particular co-stimulatory domain **5** and CD3z signaling domain **6**. RevCAR consists of the 5B9 epitope (tag **7**), connected to an extracellular hinge and transmembrane domain **4**, co-stimulatory domain **5** and CD3z signaling domain **6**, wherein the extracellular hinge domain **5** and transmembrane domain **6** of the CAR polypeptide **1** are the same or different from the switchable CAR (RevCAR) polypeptide **2**.
- **Fig. 2**: shows the expression of the CAR and RevCAR on T cells of seven different construct variants according to the invention comprising one CAR (CD19 and CD20 dual targeting CAR) and one switchable CAR, in particular RevCAR with a 5B9 epitope tag, in comparison with untransduced T cells (UTD), T cells comprising one CAR (CD19 and CD20 dual targeting CAR = CD19-CD20-CAR) and T cells comprising one switchable CAR (RevCAR). Surface detection of CAR was carried out using G₄S linker antibody and surface detection of RevCAR was carried out with the 5B9 (anti-tag) antibody.

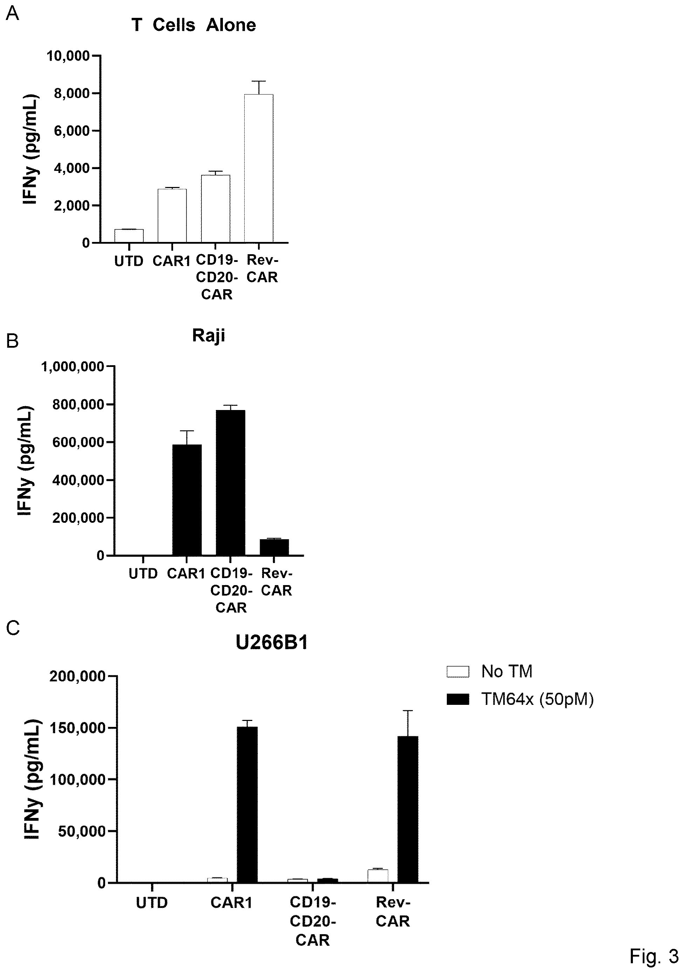

- **Fig. 3**: shows interferon gamma production of T cells according to the invention (CAR1-CAR7) comprising one CAR (CD19 and CD20 dual targeting CAR) and one switchable CAR, in particular RevCAR with a 5B9 epitope tag, alone or co-cultured with tumor cells in comparison with untransduced T cells (UTD), T cells comprising one CAR (CD19 and CD20 dual targeting CAR = CD19-CD20-CAR) and T cells comprising one switchable CAR (RevCAR). Secreted interferon-gamma was measured in co-culture supernatants after 24 hours. T cells were cultured (A) alone, (B) with CD19 and CD20 positive cell line Raji and (C) with U226B1 tumor cells with and without the addition of 50 pM targeting module comprising a tag-binding domain and a target cell-binding domain binding BCMA.

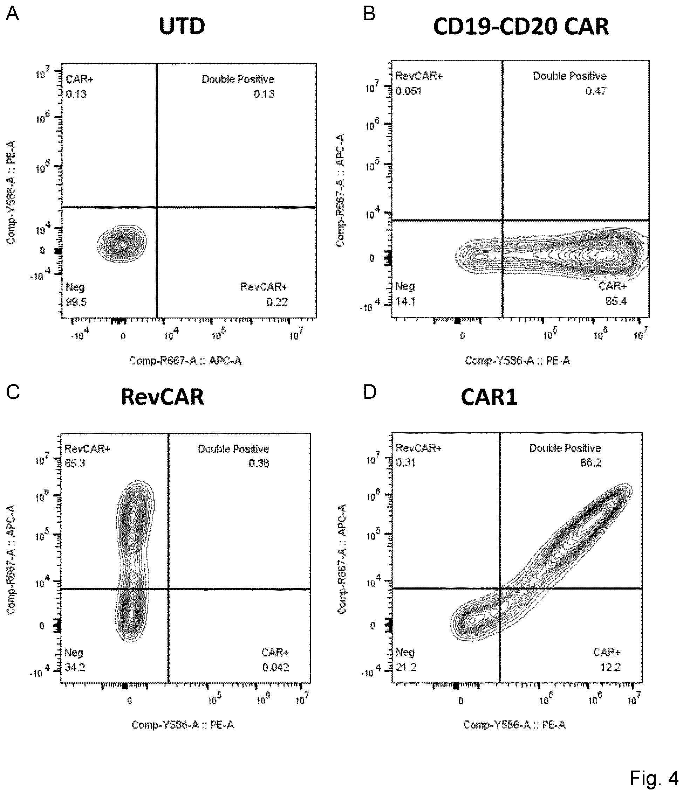

- **Fig. 4**: shows representative contour plots of CD19-CD20-CAR and RevCAR expressed on T cell surface: untransduced T cells (UTD), T cells comprising one CAR (CD19 and CD20 dual targeting CAR = CD19-CD20-CAR), T cells comprising one switchable CAR (RevCAR) and T cells according to the invention comprising one CAR (CD19 and CD20 dual targeting CAR) and one switchable CAR, in particular RevCAR with a 5B9 epitope tag. Dual stain of CAR detected with G₄S linker antibody and RevCAR detected with the 5B9 antibody.

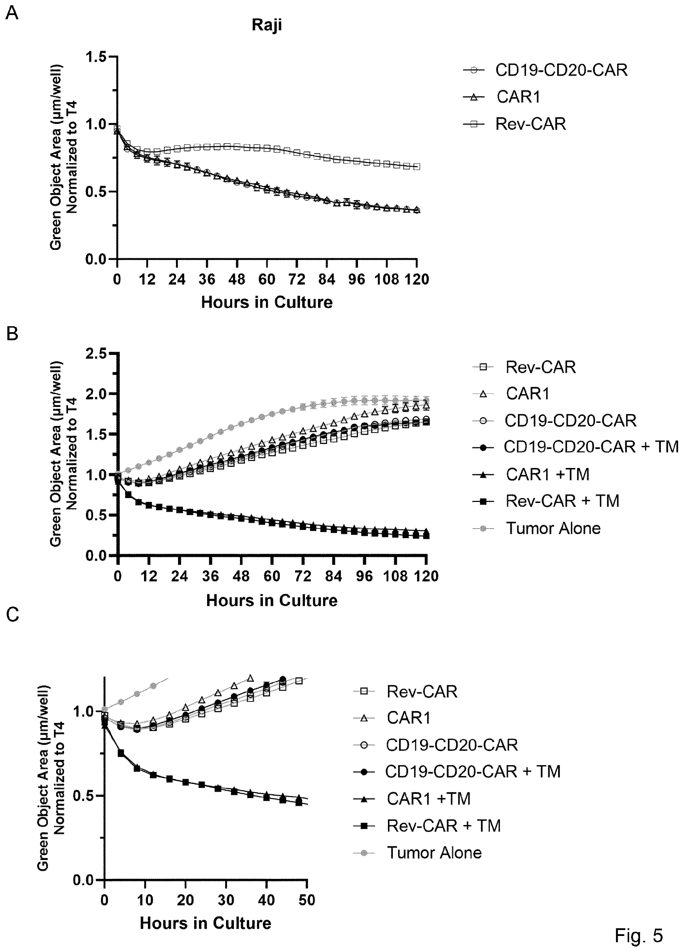

- **Fig. 5**: shows tumor cell growth and cytotoxicity of green fluorescent protein (GFP) expressing tumor cell lines co-cultured alone or with engineered T cells: (**A**) Tumor cell growth of GFP expressing Raji cells. Raji co-cultured (effector cell to tumor cell ratio (E:T) 3:1) with T cells according to the invention comprising one CAR (CD19 and CD20 dual targeting CAR) and one switchable CAR, in particular RevCAR with a 5B9 epitope tag (CAR1, black line, open triangle), in comparison with T cells comprising one CAR (CD19 and CD20 dual targeting CAR, CD19-CD20-CAR, black line, open circle), and T cells comprising RevCAR (black line, open square). (**B**) Tumor cell growth of GFP expressing U226B1 cells. U226B1 co-cultured (effector cell to tumor cell ratio (E:T) 3:1) with T cells according to the invention comprising one CAR (CD19 and CD20 dual targeting CAR) and one switchable CAR, in particular RevCAR with a 5B9 epitope tag (CAR1, grey line, open triangle) or additionally with 50 pm targeting module (TM, black line, closed triangle), in comparison with T cells comprising one CAR (CD19 and CD20 dual targeting CAR, CD19-CD20-CAR, grey line, open circle) or additionally with 50 pm targeting module (TM, black line, closed circle), and T cells comprising RevCAR (grey line, open rectangle) or additionally with 50 pm targeting module (TM, black line, closed rectangle), U226B1 cultured alone (grey line, closed circle). (**C**) focused graph of Fig. 5B first 50 hours of culture.

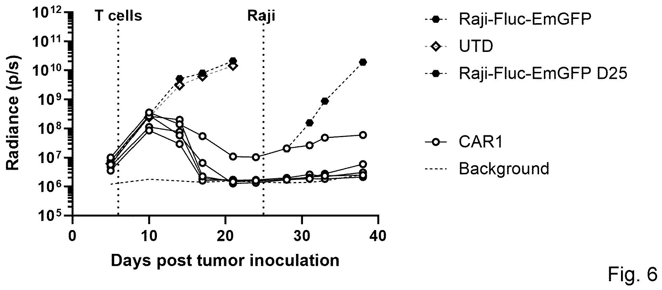

- **Fig. 6**: shows in vivo efficacy of engineered CAR T cells in B cell lymphoma CDX model: mean value and standard error of the mean (SEM) of total body radiance (p/s) for control groups (Raji-Fluc-EmGFP, untransduced T cell (UTD), Raji-Fluc-EmGFP D25, n=5 mice per groups) and individual animal data for T cells according to the invention comprising one CAR (CD19 and CD20 dual targeting CAR) and one switchable CAR, in particular RevCAR with a 5B9 epitope tag (CAR1).

### Engineered T cells comprising one chimeric antigen receptor polypeptide specific for CD19 and CD20 and one switchable chimeric antigen receptor polypeptide

For genetical engineering to express CARs and switchable CARs, a polynucleotide vector encoding the CAR and a polynucleotide vector encoding the switchable CAR and all necessary elements to ensure their expression in the genetically engineered immune cell is transferred into the immune cell. In particular, the CAR comprises a CD19-binding scFv, a CD20-binding V_{H}H, an extracellular domain, a transmembrane domain, and two intracellular domains (CD3ζ and co-stimulatory domain) and the switchable CAR comprises a tag, an extracellular hinge domain and transmembrane domain, and two intracellular domains (CD3ζ and co-stimulatory domain) (see Fig. 1), wherein the extracellular hinge domain and transmembrane domain of the CAR polypeptide are different from the switchable CAR (RevCAR) polypeptide (see Fig. 1B) or the same.

CAR1 used has SEQ ID No. 86, CAR2 SEQ ID No. 87, CAR3 SEQ ID No. 88, CAR4 SEQ ID No. 89, CAR5 SEQ ID No. 90, CAR6 SEQ ID No. 91 and CAR7 SEQ ID No. 92.

The transfer of the vectors can be performed by electroporation or transfection of nucleic acids or the help of viral vector systems like adeno-, adeno-associated, retro-, foamy- or lentiviral viral gene transfer.

Gamma retrovirus is used for delivery and stable expression of the CAR construct. 293Vec-RD114 cell lines (biovec pharma) are transfected with gamma retroviral transfer plasmid using TransIT-VirusGEN (Mirus Bio) transfection reagent. The RD114 cell line contains stable integration of the helper gene GAG and POL and glycoprotein RD114. Transfection with the transfer plasmid provides the necessary components for virus assembly. Twenty-four hours after the transfection media is replaced and cell supernatant containing virus is harvested at 48 and 72 hours and filtered through 0.45 µm filter and frozen at -80°C.

To produce transduced CAR T cells, T cells are isolated from fresh leukopaks using the Straight from Leukopak CD4+/CD8+ MicroBead kit (Miltenyi Biotec). Isolated T cells were frozen and stored in LN2 vapor phase. T cell aliquots are thawed in 1 ml of human AB serum (HABs), washed in CTS-low (CTS optimizer, 1% Glutamax, 100 IU/ml IL2, 30 IU/ml of IL-7 and IL-15) and adjusted to 1·10⁶ cells/ml and cultured with a 1:100 volumetric dilution of TransAct (Miltenyi Biotec). One million cells were cultured in a GREX24 or 5 million in a GREX6M (Wilson Wolf). Forty-eight hours after initiation, T cells are transduced with an equal volume of gamma retroviral vector and a 1:100 dilution of vectofusin (Miltenyi Biotec). Twenty-four hours later, the GREX is filled with CTS-low supplemented with 2% HABs. Cultures are harvested and cryopreserved after 6 or 10 days in culture.

Detection of the CAR is performed by staining 1 million T cells. Cells are harvested and washed in PBS and stained with Live/Dead NIR (Invitrogen) for 20 mins. Staining is quenched in Flow Cytometry Staining Buffer (eBioscience). Antibodies for CAR detection include GaS Linker (E702V, Cell Signaling) and rabbit anti-mouse FMC63 anti-idiotype (AcroBiosystems) and goat anti-mouse-fluorescently labeled secondary (BioLegend). Cells surface stains are recorded on a NovoCyte (Agilent Technologies) and analyzed in a single-cell flow cytometer (Flow-Jo^{™}).

To assess the functionality of the CAR T cells, engineered T cells are incubated with tumor cells at a 1:1 effector to T cell ratio (E:T) for 24 hours. Cell supernatants are harvested and frozen. Cytokine production (IFNg) is quantified by ELISA (Meso Scale Discovery) and plotted in Prism. To assess the cytotoxic potential of CAR T cells, engineered T cells are cultured with tumor cell lines stably expressing green fluorescent protein at various E:T ratios. Green fluorescence is tracked using the Incucyte Live Cell Imaging System for up to 5 days. Loss of green signal indicates cytotoxicity of tumor cells.

The in vivo efficacy of engineered CAR T cells was evaluated in B cell lymphoma CDX model (see Fig. 6). NSG mice were inoculated intravenously with 2×10⁵ Raji tumor cells expressing firefly luciferase and emerald green fluorescent protein (Raji-Fluc-EmGFP). Raji cells express both CD19 and CD20. At day 5, tumor engraftment was confirmed by bioluminescence imaging (BLI) using an IVIS System and mice were randomized based on total body radiance (p/s). At day 6, engineered CAR-T cells were thawed and formulated for matching CAR positive T cells dose per mouse. In this experiment, mice received 1×10⁶ CAR positive T cells intravenously though the tail vein. Control groups received no T cells or untransduced (UTD) T cell dose matching the total T cell dose of the lowest CAR-T cell group. Tumor progression was followed by bi-weekly imaging and three-time weekly body weight and body score. At day 25, surviving mice were rechallenged with 2·10⁶ Raji-Fluc-EmGFP tumor cells. A group of naive mice was also inoculated at day 25 with Raji-Fluc-EmGFP as control for tumor growth in absence CAR-T cells.

For Figs. 2 to 6 T cells have been engineered to express sequences according to SEQ ID No. 86 (CAR1), SEQ ID No. 65 or SEQ ID No. 66 (CD19-CD20-CAR) and SEQ ID No. 74 (RevCAR). The used targeting module comprises a tag-binding domain and a target cell-binding domain binding BCMA, in particular according to SEQ ID No. 100 to SEQ ID No. 102.

Fig. 2 shows that the CD19- and CD20-binding domain of the CAR and the switchable CAR are expressed on the T cell surface.

Fig. 3 shows that the CD19- and CD20-binding domain of the CAR and the switchable CAR according to the invention are functioning.

Fig. 4 shows that the CD19- and CD20-binding domain of the CAR and the switchable CAR are predominately expressed together on the same cell.

Fig. 5 shows that the CD19- and CD20-binding domain of the CAR and the switchable CAR can induce cytotoxicity of tumor cells expressing target antigens.

Fig. 6 shows that T cells comprising one CAR (CD19 and CD20 dual targeting CAR, CD19-CD20-CAR) and T cells according to the invention comprising one CAR (CD19 and CD20 dual targeting CAR) and one switchable CAR, in particular RevCAR with a 5B9 epitope tag (CAR1) both control tumor growth of B cell lymphoma expressing CD19 and CD20 and a rechallenge with 10-fold higher tumor inoculation dose. The T cells according to the invention (CAR1) outperform CD19/CD20 CAR-T cells in controlling a primary and a secondary challenge.

### Cited non-patent literature:

Brentjens RJ, Rivière I, Park JH, Davila ML, Wang X, Stefanski J, Taylor C, Yeh R, Bartido S, Borquez-Ojeda O, Olszewska M, Bernal Y, Pegram H, Przybylowski M, Hollyman D, Usachenko Y, Pirraglia D, Hosey J, Santos E, Halton E, Maslak P, Scheinberg D, Jurcic J, Heaney M, Heller G, Frattini M, Sadelain M (2011) Safety and persistence of adoptively transferred autologous CD19-targeted T cells in patients with relapsed or chemotherapy refractory B-cell leukemias. Blood 118(18):4817-4828. doi: https://doi.org/10.1182/blood-2011-04-348540
Cartellieri M, Bachmann M, Feldmann A, Bippes C, Stamova S, Wehner R, Temme A, Schmitz M (2010) Chimeric Antigen Receptor-Engineered T Cells for Immunotherapy of Cancer J. Biomed. Biotechnol. Article ID 956304, doi: 10.1155/2010/956304.Cartellieri M, Feldmann A, Koristka S, Arndt C, Loff S, Ehninger A, von Bonin M, Bejestani EP, Ehninger G, Bachmann MP (2016) Switching CAR T cells on and off: a novel modular platform for retargeting of T cells to AML blasts. Blood cancer J. 6 (8), e458.
Darowski D, Kobold S, Jost C, Klein C (2019) Combining the best of two worlds: highly flexible chimeric antigen receptor adaptor molecules (CAR-adaptors) for the recruitment of chimeric antigen receptor T cells. MAbs. 11 (4), 621-631.
Feldmann A, Hoffmann A, Bergmann R, Koristka S, Berndt N, Arndt C, Rodrigues Loureiro L, Kittel-Boselli E, Mitwasi N, Kegler A, Lamprecht C, González Soto KE, Bachmann M (2020) Versatile chimeric antigen receptor platform for controllable and combinatorial T cell therapy. Oncoimmunology. 9 (1), 1785608.
Grupp SA, Laos M, Barrett D, Aplenc R, Porter DL, Rheingold SR, Teachey DT, Chew A, Hauck B, Wright JF, Milone MC, Levine, BL, June CH (2013) Chimeric Antigen Receptor-Modified T Cells for Acute Lymphoid Leukemia. N.Engl.J.Med. 368, 1509-1518.
Kalos M, Levine BL, Porter DL, Katz S, Grupp SA, Bagg A, June CH (2011) T cells with chimeric antigen receptors have potent antitumor effects and can establish memory in patients with advanced leukemia. Sci Transl Med. 3(95):95ra73. doi: 10.1126/scitranslmed.3002842. PMID: 21832238; PMCID: PMC3393096.
Kobos R, Curran K, Steinherz P, Jurcic J, Rosenblat T, Maslak P, Frattini M, Sadelain M (2013) CD19-targeted T cells rapidly induce molecular remissions in adults with chemotherapy-refractory acute lymphoblastic leukemia. Sci Transl Med. 5(177):177ra38. doi: 10.1126/scitranslmed.3005930. PMID: 23515080; PMCID: PMC3742551.
Kershaw MH, Westwood JA, Parker LL, Wang G, Eshhar Z, Mavroukakis SA, White DE, Wunderlich JR, Canevari S, Rogers-Freezer L, Chen CC, Yang JC, Rosenberg SA, Hwu P (2006) A Phase I Study on Adoptive Immunotherapy Using Gene-Modified T Cells for Ovarian Cancer. Clin.Cancer Res. 12(20):6106-6115.
Lamers CH, Sleijfer S, Vulto AG, Kruit WH, Kliffen M, Debets R, Gratama JW, Stoter G, Oosterwijk E (2006) Treatment of metastatic renal cell carcinoma with autologous T-lymphocytes genetically retargeted against carbonic anhydrase IX: first clinical experience. J Clin Oncol. 24(13):e20-e22. doi: 10.1200/JCO.2006.05.9964. PMID: 16648493.
Morgan RA, Yang JC, Kitano M, Dudley ME, Laurencot CM, Rosenberg SA (2010) Case Report of a Serious Adverse Event Following the Administration of T Cells Transduced With a Chimeric Antigen Receptor Recognizing ERBB2. Mol. Ther. 18, 843-851.
Reinke AW, Grant RA, Keating AE (2010) A Synthetic Coiled-Coil Interactome Provides Heterospecific Modules for Molecular Engineering. JACS 132, 6025-6031.
Sotillo E, Barrett DM, Black K., Bagashev A, Oldridge D, Wu G, Sussman R, Lanauze C, Ruella M, Gazzara MR, Martinez NM, Harrington CT, Chung EY, Perazzelli J, Hofmann TJ, Maude SL, Raman P, Barrera A, Gill S, Lacey SF, Melenhorst JJ, Allman D, Jacoby E, Fry T, Mackall C, Barash Y, Lynch KW, Maris JM, Grupp SA, Thomas-Tikhonenko A (2015) Convergence of Acquired Mutations and Alternative Splicing of CD19 Enables Resistance to CART-19 Immunotherapy. Cancer Discov. 5:1282-1295.
Smith TF, Waterman MS (1981) Identification of common molecular subsequences. J Mol Biol. 147(1):195-197. doi: 10.1016/0022-2836(81)90087-5.
Weber EW, Parker KR, Sotillo E, Lynn RC, Anbunathan H, Lattin J, Good Z, Belk JA, Daniel B, Klysz D, Malipatlolla M, Xu P, Bashti M, Heitzeneder S, Labanieh L, Vandris P, Majzner RG, Qi Y, Sandor K, Chen LC, Prabhu S, Gentles AJ, Wandless TJ, Satpathy AT, Chang HY, Mackall CL (2021) Transient rest restores functionality in exhausted CAR-T cells through epigenetic remodeling. Science 372 (6537), eaba1786.

### Reference signs

- **1**: CD20 binding domain
- **2**: linker
- **3**: CD19 binding domain
- **4**: extracellular hinge and transmembrane domain
- **5**: co-stimulatory domain
- **6**: signaling domain
- **7**: tag-binding domain or tag

## Claims

1. An engineered cell comprising:
a) at least one chimeric antigen receptor (CAR) polypeptide comprising:
• at least one target cell-binding domain,
• a first extracellular hinge domain,
• a first transmembrane domain, and
• at least one first intracellular signaling domain,
b) at least one switchable chimeric antigen receptor (CAR) polypeptide comprising:
• a tag-binding domain or a tag,
• a second extracellular hinge domain,
• a second transmembrane domain, and
• at least one second intracellular signaling domain.

2. The engineered cell of claim 1, wherein the at least one CAR polypeptide is specific for CD19 and CD20 and comprises at least one CD19-binding domain and at least one CD20-binding domain.

3. The engineered cell of claim 2, wherein the at least one CD19-binding domain comprises a heavy chain variable region having complementary-determining regions (CDRs) with an amino acid sequence according to SEQ ID No. 1 (CDR-H1), an amino acid sequence according to SEQ ID No. 2 (CDR-H2), and an amino acid sequence according to SEQ ID No. 3 (CDR-H3), and a light chain variable region having CDRs with an amino acid sequence according to SEQ ID No. 4 (CDR-L1), an amino acid sequence HTS (CDR-L2), and an amino acid sequence according to SEQ ID No. 5 (CDR-L3), and/or a sequence with an identity of at least 95 % with the amino acid sequence according to SEQ ID No. 6, and/or the at least one CD20-binding domain comprises a heavy chain variable region having CDRs with an amino acid sequence according to SEQ ID No. 7 (CDR-H1), an amino acid sequence according to sequence SEQ ID No. 8 (CDR-H2), and an amino acid sequence according to SEQ ID No. 9 (CDR-H3), and/or a sequence with an identity of at least 95 % with the amino acid sequence according to SEQ ID No. 10.

4. The engineered cell of any one of claims 1 to 3, wherein the first and the second extracellular hinge domain are independently from each other selected from the group consisting of an extracellular hinge domain of CD4, CD8α, CD28, ICOS (CD278), IgG1, IgG2, IgG4 or mutants thereof.

5. The engineered cell of any one of claims 1 to 4, wherein the first and the second transmembrane domain are independently from each other selected from the group consisting of a transmembrane domain of CD4, CD8α, CD28, ICOS (CD278), IgG1, IgG2, IgG4 or mutants thereof.

6. The engineered cell of any one of claims 1 to 5, wherein the at least one first and the at least one second intracellular signaling domain are independently from each other selected from the group consisting of a cytoplasmic domain of CD3, CD27, CD28, OX40 (CD134), 4-1BB (CD137), ICOS (CD278), DAP10, DAP12, PD-1, CTLA-4, IL-2 receptor, IL-7 receptor, IL-15 receptor or IL-21 receptor and mutants thereof.

7. The engineered cell of any one of claims 1 to 6, wherein the tag is a peptide epitope tag, preferably a myc-tag, a His-tag, a linear peptide sequence from yeast transcription factor GCN4, a leucine zipper sequence or a peptide sequence from a human nuclear protein, preferably from the human La protein.

8. The engineered cell of any one of claims 1 to 7, wherein the tag-binding domain is an antibody, antigen-binding fragment, a protein or a peptide binding to a myc-tag, a His-tag, a peptide sequence from yeast transcription factor GCN4, a leucine zipper sequence or a peptide sequence from a human protein.

9. The engineered cell of any one of claims 1 to 8, wherein the at least one CAR polypeptide specific for CD19 and CD20 comprises one of the sequences selected from SEQ ID No. 65 to SEQ ID No. 67 or a sequence with an identity of at least 95 % with one of the sequences selected from SEQ ID No. 65 to SEQ ID No. 67 and/or the at least one switchable CAR polypeptide comprises one of the sequences selected from SEQ ID No. 74 to SEQ ID No. 79 or a sequence with an identity of at least 95 % with one of the sequences selected from SEQ ID No. 74 to SEQ ID No. 79.

10. The engineered cell of any one of claims 1 to 9 for use in the treatment of cancer, infectious disease or autoimmune disease.

11. A pharmaceutical composition comprising the engineered cell of any one of claims 1 to 9 and a pharmaceutically acceptable thinner or carrier.

12. A kit comprising the at least one engineered cell of any one of claims 1 to 8 and/or
i) at least one first nucleic acid or vector comprising a nucleotide sequence encoding the at least one CAR polypeptide comprising:
• at least one target cell-binding domain,
• a first extracellular hinge domain,
• a first transmembrane domain, and
• at least one first intracellular signaling domain, and
ii) at least one second nucleic acid or vector comprising a nucleotide sequence encoding the at least one switchable CAR polypeptide comprising:
• a tag-binding domain or a tag,
• a second extracellular hinge domain,
• a second transmembrane domain, and
• at least one second intracellular signaling domain, and/or
at least one nucleic acid or vector comprising a nucleotide sequence encoding the at least one CAR polypeptide and the at least one switchable CAR polypeptide.

13. The kit of claim 12, wherein the kit further comprises at least one targeting module comprising at least one further target cell-binding domain capable of binding a target antigen and a tag or tag-binding domain and/or at least one nucleic acid, vector and/or cell encoding a targeting module,
wherein the further target cell-binding domain is different from the target cell-binding domain of the CAR polypeptide,
wherein the tag-binding domain of the targeting module binds to the tag of the switchable CAR or the tag of the targeting module binds to the tag-binding domain of the switchable CAR.

14. The kit of claim 13, wherein the target cell-binding domain of the CAR and the further target cell-binding domain of the targeting module are independently from each other selected from an antibody, antigen-binding fragment, a protein, a peptide or a low molecular weight organic ligand that binds to a surface antigen selected from the group consisting of CD2, CD3, CD4, CD5, CD7, CD8, CD10, CD15, CD19, CD20, CD22, CD23, CD25, CD30, CD33, CD38, CD44, CD44v6, CD52, CD66a, CD66b, CD66c, CD66d, CD66e, CD66f, CD79a, CD79b, CD90, CD99, CD123, CD133, CD135, CD150, CD181, CD182, CD184, CD223, CD229, CD269, CD273, CD274, CD276, CD279, CD319, CD366, CD371, a cytokine receptor, CXCR4, c-Met, mesothelin, a member of the epidermal growth factor receptor family or a mutant thereof, a member of the tumor necrosis factor receptor superfamily, a claudin, an ephrin, an ephrin receptor, a fucosyl transferase, a prostate specific antigen, an embryonic antigen, a member of the vascular endothelia growth factor family, epithelial cell adhesion molecule, alpha-fetoprotein, a member of the intercellular adhesion molecule family, a C-type lectin, an integrin, a member of the mucin protein family, a follicle-stimulating hormone receptor, a high molecular weight-melanoma associated antigen, a folate binding protein, a folate receptor, a somatostatin receptor, a ligand of the NKG2D receptor, a member of the epithelia glycoprotein family, a disialoganglioside, a glypican, a G protein-coupled receptor, a human papillomavirus protein, cancer/testis antigen, fibroblast activation protein, a member of the carbonic anhydrase family, a member of the carbohydrate antigen family, a Notch ligand, melanoma-associated chondroitin sulfate proteoglycan, glycoprotein A33, guanylate cyclase 2C and tumor-specific glycan.

15. The kit of claim 13 or 14, wherein the length of the targeting module is in the range of 20 to 1600 amino acids, preferably 200 to 1450 amino acids.

16. The kit of any one of claims 12 to 15, wherein the first and/or second vector are independently from each other selected from the group consisting of a DNA vector, an RNA vector, a plasmid, a lentiviral vector, retroviral vector, adenoviral vector and an adeno-associated viral vector.

17. The kit of any one of the claims 11 to 15, wherein the kit comprises at least one engineered cell and at least one targeting module, for use in the treatment of cancer, infectious disease or autoimmune disease.
